# EUROPÄISCHE PATENTSCHRIFT

(11) **EP 1 489 062 B1**
(45) Veröffentlichungstag und Bekanntmachung des Hinweises auf die Patenterteilung: **19.12.2012**
(21) Anmeldenummer: 04101656.9
(22) Anmeldetag: 21.04.2004
(51) Int. Cl.: C07C 1/24, C07C 11/09, B01J 8/00, B01J 8/04, B01J 8/02, B01J 8/06, B01J 19/00, B01J 19/24

(54) **Verfahren zur Herstellung von Isobuten aus tert.-Butanol**
Process for preparing isobutene from tertiary butanol
Procédé de préparation d'isobutène à partir de tertiaire butanol

(30) Priorität: 17.06.2003 DE 10327215
(43) Veröffentlichungstag der Anmeldung: 22.12.2004
(73) Patentinhaber: Evonik Oxeno GmbH, 45772 Marl (DE)
(72) Erfinder: Beckmann, Andreas Dr., 45657 Recklinghausen (DE); Büschken, Wilfried Dr., 45721 Haltern am See (DE); Grund, Gerda Dr., 48249 Dülmen (DE); Reusch, Dieter Dr., 45772 Marl (DE)
(74) Vertreter: Hirsch, Hans-Ludwig

(56) Entgegenhaltungen:
- DE-A- 3 151 446
- DE-A- 19 936 675

## Beschreibung

Die vorliegende Erfindung betrifft ein Verfahren zur Herstellung von Isobuten durch Spaltung von tert.-Butanol (TBA) an saueren Ionenaustauscherharzen.

Isobuten ist ein Ausgangsstoff für die Herstellung von Butylkautschuk, Polyisobutylen, Isobuten-Oligomeren, verzweigten C₅-Aldehyden und C₅-Carbonsäuren. Weiterhin wird es als Alkylierungsmittel und als Zwischenprodukt zur Erzeugung von Peroxiden eingesetzt.

In technischen Strömen liegt Isobuten zusammen mit gesättigten und ungesättigten C₄-Kohlenwasserstoffen vor. Aus diesen Gemischen kann Isobuten wegen der geringen Siedepunktsdifferenz bzw. des sehr geringen Trennfaktors zwischen Isobuten und 1-Buten durch Destillation nicht wirtschaftlich abgetrennt werden. Daher wird Isobuten aus technischen Kohlenwasserstoffgemischen dadurch gewonnen, dass Isobuten zu einem Derivat umgesetzt wird, das sich leicht vom übrig gebliebenden Kohlenwasserstoffgemisch abtrennen lässt, und dass das isolierte Derivat zu Isobuten und Derivatisierungsmittel zurückspaltet wird.

Üblicherweise wird Isobuten aus C₄-Schnitten, beispielsweise der C₄-Fraktion eines Steamcrackers, wie folgt abgetrennt. Nach Entfernung des größten Teils der mehrfach ungesättigten Kohlenwasserstoffe, hauptsächlich Butadien, durch Extraktion(-sdestillation) oder Selektivhydrierung zu linearen Butenen wird das verbleibende Gemisch (Raffinat 1 oder hydriertes Crack-C₄) mit Alkohol oder Wasser umgesetzt. Bei der Verwendung von Methanol entsteht aus Isobuten Methyl-tert.-butylether (MTBE) und bei Einsatz von Wasser tert.-Butanol (TBA). Nach ihrer Abtrennung können beide Produkte in Umkehrung ihrer Bildung zu Isobuten gespalten werden.

Die Spaltung von TBA lässt sich leichter als die Spaltung von MTBE durchführen und ergibt weniger Nebenprodukte. Die säurekatalysierte Spaltung von TBA zur Gewinnung von Isobuten hoher Reinheit ist bekannt. Diese Spaltung kann in der Gas- oder Flüssigphase durchgeführt werden.

Die Dehydratisierung von TBA in der Gasphase wird an saueren Alumosilikatkatalysatoren, wie beispielsweise in EP 0 255 948 beschrieben, oder an Aluminiumoxiden, wie beispielsweise in US 3 665 048 beschrieben, oder an Zeolithen, wie beispielsweise in WO 93/21139 beschrieben, durchgeführt. Der Nachteil dieser Verfahren liegt darin, dass wegen der verfahrensbedingten hohen Temperaturen und hohen Isobutenkonzentrationen Nebenprodukte durch Dimerisierung bzw. Oligomerisierung des gebildeten Isobutens entstehen. Durch Herabsetzung der Isobuten-Konzentration im dampfförmigen Edukts durch Zusatz von Inertgas versucht man, diese Nebenreaktionen zu minimieren. Dadurch entsteht ein zusätzlicher Arbeitsaufwand.

Für die Dehydratisierung von TBA in flüssiger Phase sind mehrere Verfahren bekannt. In DE 29 53 583 wird die Dehydratisierung von TBA in einer Kolonne durchgeführt, deren unterer Teil mit Füllkörpern und deren oberer Teil mit einem saueren Ionenaustauscherharz gefüllt ist. Eine wässrige TBA-Lösung wird kontinuierlich in die Kolonne knapp unter dem Ionenaustauscherharz eingespeist. Als Kopfprodukt wird ein Isobuten-haltiger Strom gewonnen, dessen Rektifikation in einer weiteren Kolonne Isobuten in 99,95 % Reinheit ergibt. Der Umsatz des TBA beträgt 99 %. Die Isobutenausbeute ist nicht offengelegt.

In DE 29 13 796 wird die TBA-Spaltung in einem Rührkessel, in dem ein saueres Ionenaustauscherharz in einer Lösung aus TBA und Wasser suspendiert ist, im Druckbereich von 5 bis 7 bar und im Temperaturbereich von 110 bis 120 °C durchgeführt. In die Flüssigphase dieses Reaktors wird ein Gemisch aus Wasser und TBA gasförmig eingeleitet. Am Kopf des Reaktor entweicht ein gasförmiges Gemisch, bestehend aus Isobuten, TBA und Wasser. Am Bodenauslass wird ein flüssiges Gemisch, bestehend aus Wasser, TBA und Nebenprodukten, standgeregelt abgezogen. Das Kopfprodukt wird destillativ in Isobuten und in ein Gemisch aus Wasser und TBA getrennt, das in den Reaktor zurückgeführt wird. Die aus dem Reaktor flüssig abgezogene Phase wird in mindestens zwei Kolonnen in Wasser, Nebenprodukte, wie Oligomere des Isobutens und TBA, das in den Reaktor zurückgeleitet wird, aufgetrennt. Neben der Komplexität der Anlage ist nachteilig, dass durch die mechanische Belastung der Katalysator geschädigt wird, was eine geringe Standdauer bedingt.

In DE 31 51 446 wird die Rückspaltung von TBA in Isobuten und Wasser an einem in einem Festbett angeordneten stark saueren Ionenaustauscherharz in homogener und flüssiger Phase bei Temperaturen von 80 °C bis 150 °C und Drücken von 5 bis 25 bar durchgeführt, und der homogene, flüssige Reaktionsaustrag in einer oder mehreren Kolonnen in Isobuten, Nebenprodukte, Wasser und ein Wasser-TBA-Gemisch, das in den Reaktor zurückgeführt wird, getrennt. Dabei enthält das gesamte in den Reaktor rückgeführte TBA mehr Wasser, als dem tert.-Butanol-Wasser-Azeotrop entspricht. Bedingt durch die Rückführung einer großen Wassermenge ist die Raum-Zeit-Ausbeute des Verfahrens nicht besonders hoch und ein hoher Energieaufwand für die Stofftrennung nötig. Zudem bedeuten die großen Kreislaufmengen außerdem einen sehr großen apparativen Aufwand bzw. eine entsprechend große Dimensionierung der Apparaturen.

Da die bekannten Verfahren hinsichtlich der Selektivität, Raumzeitausbeute, Energieaufwand und/oder Investment nicht zufriedenstellend waren, bestand die Aufgabe ein Verfahren zu entwickeln, das diese Nachteile nicht aufweist.

Es wurde nun überraschenderweise gefunden, dass das Kreislaufverhältnis und damit der apparative Aufwand sowie der Energieaufwand für die Bildung von Isobuten aus tert.-Butanol dadurch erniedrigt werden kann, dass der Reaktor in dem die Spaltung durchgeführt wird isotherm oder zumindest quasi isotherm betrieben wird.

Gegenstand der vorliegenden Erfindung ist deshalb ein Verfahren zur Herstellung von Isobuten durch Spaltung von tert-Butanol in zumindest einem Reaktor an einem in diesem Reaktor im Festbett angeordneten stark saueren Ionenaustauscherharz zu Isobuten und Wasser bei einer Temperatur von 80 bis 150 °C und einem Druck von 5 bis 25 bar und anschließende Auftrennung des Reaktionsgemisches in Isobuten, Nebenprodukte, Wasser und zumindest eine Mischung aus nicht gespaltenem tert.-Butanol und Wasser,
dadurch gekennzeichnet,
dass der Reaktor quasi isotherm mit einer Temperaturdifferenz zwischen den ein- und austretenden Strömen von weniger als 15 K betrieben wird, und
das Verfahren so durchgeführt wird, dass die Spaltung in der ersten Reaktionszone bis zum Gleichgewicht der Spaltungsreaktion durchgeführt wird, in einer anschließenden Abtrennzone Isobuten gasförmig abgetrennt wird und in einer sich daran anschließenden Reaktionszone die Spaltung wieder bis zum Gleichgewicht der Spaltungsreaktion durchgeführt wird, und, wenn weitere Abtrennzonen und Reaktionszonen vorhanden sind, diese Verfahrensschritte entsprechend häufig wiederholt werden.
quasi isotherm bedeutet dass die Temperaturdifferenz zwischen den ein- und austretenden Strömen (Edukt- und Produktstrom) weniger als 15, bevorzugt weniger als 10 K und besonders bevorzugt weniger als 5 K und ganz
besonders bevorzugt weniger als 1 K beträgt. Der Zulauf zum Reaktor setzt sich aus frischem Eduktstrom und einem oder mehreren Rückfühnmgsströmen, die eine Mischung aus nicht gespaltenem tert.-Butanol und Wasser aufweisen, zusammen, wobei das Massen-Verhältnis von frischem Eduktstrom zu der Summe der Rückführungsströme kleiner 1 : 10, bevorzugt kleiner 1 : 5 und ganz besonders bevorzugt kleiner 1 : 3 beträgt.

Außerdem ist Gegenstand der vorliegenden Erfindung ein Reaktorsystem zur isothermen Durchführung von Gleichgewichtsreaktionen in der Flüssigphase an Festbettkatalysatoren, bei denen zumindest eines der Reaktionsprodukte unter Temperatur und Druckbedingungen, bei denen alle Edukte sowie zumindest ein Reaktionsprodukt überwiegend flüssig vorliegen, zum Teil, vorzugsweise zum überwiegenden Teil, gasförmig vorliegt, insbesondere zur Durchführung des erfindungsgemäßen Verfahrens, welcher dadurch gekennzeichnet ist, dass das Reaktorsystem zumindest zwei baulich voneinander getrennte Reaktionszonen aufweist, die jeweils einen im Festbett angeordneten Katalysator sowie Vorrichtungen zur isothermen Temperaturführung aufweisen, wobei zwischen zwei Reaktionszonen jeweils eine Abtrennzone vorhanden ist, die Mittel aufweist, mit denen zumindest ein Teil zumindest eines der Produkte der Gleichgewichtsreaktion durch Druck oder Temperaturänderung in die Gasphase überführt, vom Reaktionsgemisch abgetrennt und aus dem Reaktor ausgeschleust wird und mit denen das restliche Reaktionsgemisch in die folgende Reaktionszone überführt wird, wobei gegebenenfalls ein weiteres Mittel vorhanden sein kann, mit welchem die Temperatur oder der Druck in der folgenden Reaktionszone genau so eingestellt wird wie in der vorangegangenen Reaktionszone. Dieser Reaktor oder dieses Reaktorsystem kann insbesondere für die Durchführung von endothermen Reaktionen, wie beispielsweise die Spaltung von tertiären Ethern zu den entsprechenden Isoolefmen und Alkoholen, genutzt werden.

Durch das erfindungsgemäße Verfahren ist es möglich, bei gleich hoher Selektivität der Spaltung von tert.-Butanol, mit deutlich kleineren Stoffströmen, insbesondere kleineren im Kreis geführten Stoffmengen zu operieren. Auf diese Weise können der Energiebedarf und die Dimensionen der Apparaturen gegenüber herkömmlichen Verfahren gemäß dem Stand der Technik verringert werden.

Durch die Durchführung des Verfahrens in einer speziellen Ausführungsart der Gestalt, dass mehrere Reaktoren oder Reaktionszonen in Reihe geschaltet vorhanden sind, wobei zwischen diesen Reaktionszonen, in denen die Spaltung in homogener flüssiger Phase durchgeführt wird, jeweils eine Abtrennzone vorhanden ist, in der Isobuten verdampft und gasförmig aus dem Reaktionsgemisch abgetrennt wird, ist es einfach möglich, die Spaltung über das Gleichgewichtsverhältnis hinaus durchzuführen, da in den Abtrennzonen ein Teil des Isobutens aus dem Gleichgewicht entfernt wird.

Durch die in der bevorzugten Ausführungsart des erfindungsgemäßen Verfahrens bei der TBA-Spaltung eingesetzte Entfernung von Wasser aus dem Reaktoraustrag bzw. aus anderen Wasser aufweisenden Strömen mittels einer Membran können die zu behandelnden Stoffströme weiter reduziert werden. Auf diese Weise lassen sich die Umsätze pro Reaktordurchlauf weiter steigern.

Das erfindungsgemäße Verfahren wird nachfolgend beschrieben, ohne dass das Verfahren auf diese Ausführungsformen beschränkt sein soll.

Das erfindungsgemäße Verfahren zur Herstellung von Isobuten durch Spaltung von tert.-Butanol in zumindest einem Reaktor an einem in diesem Reaktor im Festbett angeordneten stark saueren Ionenaustauscherharz zu Isobuten und Wasser, vorzugsweise bei einer Temperatur von 80 bis 150 °C und einem Druck von 5 bis 25 bar und anschließende Auftrennung des Reaktionsgemisches in Isobuten, Nebenprodukte, Wasser und zumindest eine Mischung aus nicht gespaltenem tert.-Butylalkohol und Wasser zeichnet sich dadurch aus, dass der Reaktor quasi isotherm mit einer Temperaturdifferenz zwischen den ein und austretenden Strömen (Eduktund Produktstrom) von weniger als 15, bevorzugt weniger als 10 K und besonders bevorzugt weniger als 5 K und ganz besonders bevorzugt weniger als 1 K betrieben wird. Der Zulauf zum Reaktor setzt sich aus frischem Eduktstrom und einem oder mehreren Rückführungsströmen, die eine Mischung aus nicht gespaltenem tert.-Butanol und Wasser aufweisen, zusammen, wobei das Massen-Verhältnis von frischem Eduktstrom zu der Summe der Rückführungsströme kleiner 1 : 10, bevorzugt kleiner 1 : 5 und ganz besonders bevorzugt kleiner 1 : 3 beträgt. Es kann vorteilhaft sein, wenn mehrere Reaktoren, die in Reihe oder parallel angeordnet sind, eingesetzt werden, wobei vorzugsweise alle Reaktoren isotherm betrieben werden. Es können verschiedene Reaktortypen, in denen Ionenaustauscherharz als Festbett vorliegen kann, eingesetzt werden, wie beispielsweise Festbettreaktoren, Rohrbündelreaktoren oder Reaktoren anderer Bauart. Die eingesetzten Reaktoren müssen Mittel zur isothermen Fahrweise, also zum Ab- oder Zuführen von Wärme, wie z. B. Wärmetauscher aufweisen. Der/die Reaktor(en) wird/werden quasi isotherm, d. h. dass die Temperaturdifferenz zwischen den ein- und austretenden Strömen (Edukt- und Produktstrom) weniger als 15, bevorzugt weniger als 10 K beträgt, vorzugsweise im geraden Durchgang betrieben und können von oben nach unten oder umgekehrt durchströmt werden.

Die Reaktionstemperatur in dem/den Spaltungsreaktor(en) liegt beim erfindungsgemäßen Verfahren bevorzugt im Bereich von 80°C bis 150 °C, vorzugsweise im Bereich von 100°C bis 130 °C. Bei Verwendung mehrerer Reaktoren können die Temperaturen unabhängig voneinander gleich oder verschieden sein.

Der Reaktionsdruck im Spaltungsreaktor bzw. in den Spaltungsreaktoren beträgt von 5 bis 25 bar, wobei der Druck so hoch gawählt wird, dass das in den Reaktoren, bzw. in den Reaktionszonen der Reaktoren entstehende Isobuten ohne Ausbildung einer Gasphase nahezu vollständig und im wesentlichen homogen im Reaktionsgemisch gelöst bleibt. Im Allgemeinen ist es zweckmäßig, wenn der Druck im Spaltungsreaktor größer ist als der Kolonnendruck an der Einspeisestelle einer dem Reaktor bzw. dem letzten Reaktor einer Reaktorkaskade nachgeschalteten Kolonne zur Abtrennung des erhaltenen Isobutens aus dem Produktstrom, weil dann zur Unterstützung der Isobutenabtrennung die Entspannungsverdampfung genutzt werden kann und eine Förderpumpe eingespart werden kann.

Bevorzugt wird das Verfahren in einem oder mehreren Reaktoren durchgeführt, der bzw. die einen stückigen im Festbett angeordneten saueren Ionenaustauscher als Katalysator aufweist/aufweisen, durchgeführt.

Eine bevorzugte Gruppe von als Katalysatoren eingesetzten sauren Ionenaustauscherharzen Katalysatoren sind feste Ionenaustauscherharze mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, Lewatit K2611, Lewatit K2631, OC 1501, Lewatit K2671, Lewatit K2629, Lewatit K2431.

Die Eigenschaften dieser Harze, insbesondere spezifische Oberfläche, Porosität, Stabilität, Quellung bzw. Schrumpfung und Austauschkapazität, können durch den Herstellprozess variiert werden.

Optional können auch kommerzielle, makroporöse Kationenaustauscher, die durch partiellen Ionenaustausch oder durch thermische Desulfonierung modifiziert sind, eingesetzt werden.

Im erfindungsgemäßen Verfahren werden die Ionenaustauscherharze bevorzugt in ihrer H-Form verwendet. Die Ionenaustauscherkapazität beträgt vorzugsweise von 2 bis 7, insbesondere 3 bis 6 eq/kg (bezogen auf feuchtes handelsübliches Harz).

Es werden besonders bevorzugt makroporöse Harze eingesetzt, wie beispielsweise Lewatit SCP 118 , Lewatit SCP 108, Lewatit K2631, Amberlyst 15 oder Amberlyst 35. Optional können die Ionenaustauscherharze als Formkörper, wie beispielsweise Zylinder, Ringe oder Kugeln, eingesetzt werden.

Die Korngröße der technischen Harze liegt im Allgemeinen zwischen 0,5 und 2 mm. Die Korngrößenverteilung kann aber auch enger oder weiter gewählt werden. So können beispielsweise auch Ionenaustauscherharze mit sehr einheitlicher Korngröße (monodisperse Harze) eingesetzt werden.

Es kann vorteilhaft sein, in Reaktoren, die mit hohen Lineargeschwindigkeiten durchströmt werden, zur Verringerung des Differenzdruckes ein größeres Korn einzusetzen, und in Reaktoren, die mit einer geringen Lineargeschwindigkeit durchströmt werden, zur Erzielung des optimalen Umsatzes ein kleineres Korn einzusetzen.

Bei der Verwendung mehrerer Reaktoren können diese mit Harz gleicher oder unterschiedlicher Korngröße (bzw. Korngrößenverteilung) oder mit unterschiedlichen oder gleichen Formkörpern bestückt sein.

Um zu verhindern, dass das Harz während des Betriebs saure Gruppen abspaltet und somit Störungen im Aufarbeitungsteil des Verfahren verursachen werden könnten und um eine hohe Aktivität des Katalysators über einen möglichst langen Zeitraum beizubehalten, kann das Ionenaustauscherharz vorbehandelt werden, beispielsweise durch Spülen mit Wasser, dem TBA oder TBA/Wasser-Gemischen. Das Spülen wird vorzugsweise bei einer Temperatur von 40 bis 120 °C durchgeführt.

Da der Umsatz im Reaktor meist durch das chemische Gleichgewicht und/oder die Mischungslücke begrenzt ist, sind oft große Kreislaufströme erforderlich, um das TBA in einer Anlage nahezu vollständig zu spalten. Um die Kreisläufe bei der Aufarbeitung des Reaktoraustrags möglich gering zu halten bzw. zu verringern ist es vorteilhaft, besondere Bauarten von Reaktoren oder Reaktorsysteme einzusetzen, die eine Reaktionsführung erlauben, die diese Nachteile vermeiden. Besonders bevorzugt wird das Verfahren deshalb so durchgeführt, dass die Spaltung in einer ersten Reaktionszone bis (nahe) zum Gleichgewicht der Spaltungsreaktion durchgeführt wird, in einer anschließenden Abtrennzone Isobuten gasförmig abgetrennt wird und in einer sich daran anschließenden Reaktionszone die Spaltung wieder bis (nahe) zum Gleichgewicht der Spaltungsreaktion durchgeführt wird, und, wenn weitere Abtrennzonen und Reaktionszonen vorhanden sind, diese Verfahrensschritte entsprechend häufig wiederholt werden. Durch diese Maßnahme kann erreicht werden, dass die Spaltungsreaktion nicht nur bis zur einmaligen Einstellung des Gleichgewichts erfolgt, sondern in Abhängigkeit von der Anzahl der Abtrennzonen die Reaktion mehrfach bis zur Einstellung des Reaktionsgleichgewichts durchgeführt wird. Auf diese Weise wird durch einen einzigen Durchlauf des Reaktionsgemisches durch den Reaktor bzw. das Reaktorsystem eine weitergehende Spaltung als bei herkömmlichen Reaktoren bzw. Reaktorsystemen erreicht.

Die vorgenannte Verfahrensweise kann auf verschiedene Arten erzielt werden. So kann es z. B. vorteilhaft sein, in dem erfindungsgemäßen Verfahren zumindest einen Reaktor einzusetzen, der zumindest zwei Reaktionszonen aufweist, die jeweils ein im Festbett angeordneten stark saueres Ionenaustauscherharz aufweisen, wobei zwischen zwei Reaktionszonen jeweils eine Abtrennzone vorhanden ist, in der zumindest ein Teil eines der Produkte der Spaltung durch (teilweise) Überführung in die Gasphase vom Reaktionsgemisch abgetrennt wird und aus dem Reaktor ausgeschleust wird. In den Reaktionszonen sind jeweils Mittel, wie z. B. beheizbare Platten oder Rohre vorhanden, mit welchen die isotherme Betriebsführung zumindest in den Reaktionszonen gewährleistet werden kann. Der Druck und die Temperatur in den Reaktionszonen des Reaktors wird vorzugsweise so gewählt, dass das Reaktionsgemisch in flüssiger Phase vorliegt. Der Druck und die Temperatur in den Abtrennzonen wird vorzugsweise so gewählt, dass das Isobuten zumindest zum Teil in der Gasphase vorliegt.

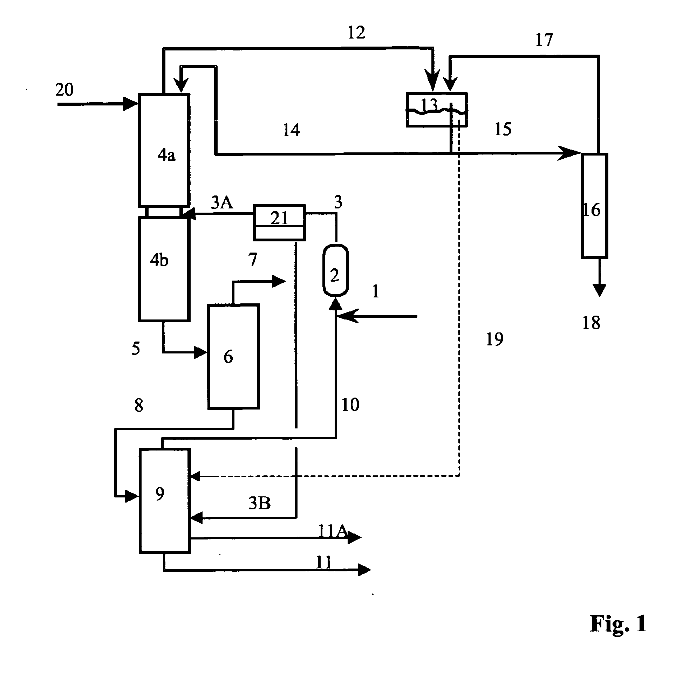

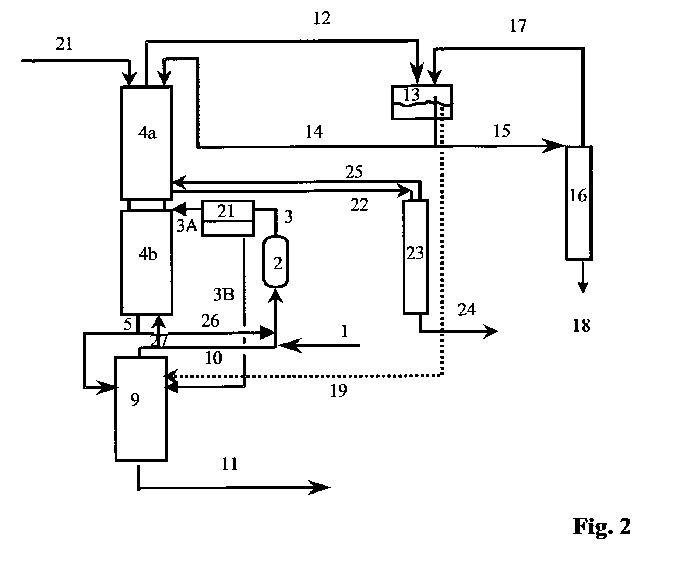

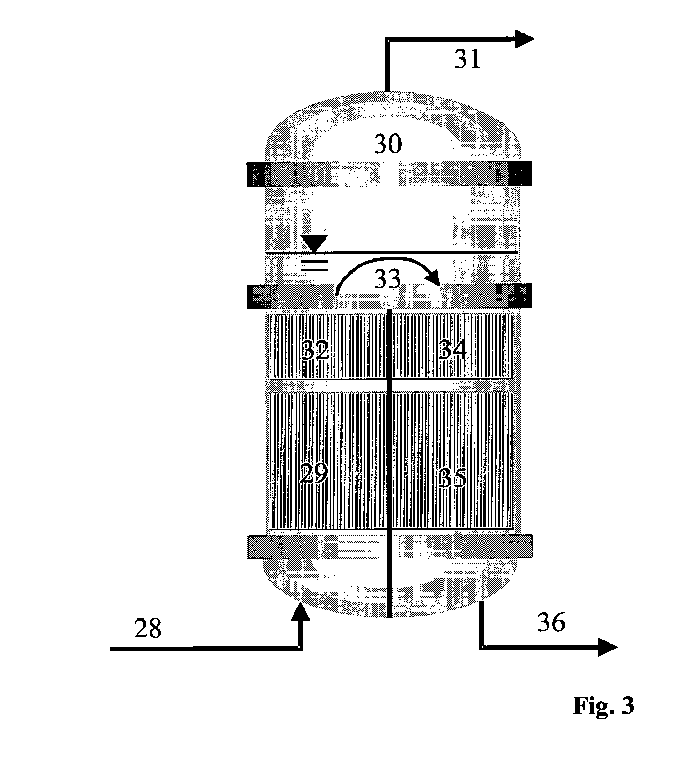

Fig. 3 zeigt den Aufbau eines solchen besonderen Reaktors. Der Reaktorzulauf (28) wird dem Reaktor von unten zugeführt und in den ersten Reaktionsbereich (29) geleitet. Dort befindet sich Katalysator in beheizbaren Rohren oder anderen beheizbaren Zwischenräumen. Durch die ablaufende Reaktion entsteht Isobuten und Wasser, bis maximal zum Erreichen des chemischen Gleichgewichts. Durch geschickte Wahl des Betriebsdrucks des Reaktors verdampft ein Teil des Isobutens und bildet eine Dampfphase (30), welche als Produktstrom (31) direkt in Kolonne (4) der Fig. 1 oder 2 geleitet werden kann. Das Verdampfen des Isobutens kann noch zusätzlich durch ein optionales Heizregister (32) verstärkt werden. Die flüssige Phase (33) kann mit einem weiteren Wärmetauscherelement (34) wieder auf die zulässige Reaktionstemperatur eingestellt werden, bevor sie in den zweiten Reaktionsbereich (35), welcher ebenfalls wie der erste (29) mit Katalysator befüllt und beheizt werden kann, geleitet wird. Dort läuft die Reaktion wieder weiter bis maximal zum chemischen Gleichgewicht. Die Reaktionsbereiche (29) und (35) sind baulich voneinander getrennt. Die austretenden Teilströme (36) und (31) entsprechen in Summe dem Strom (3) in Fig. 1 und 2. Während Strom (31) direkt in die Kolonne (4) eingeleitet wird, kann Strom (36) wie in Fig. 1 und 2 gezeigt erst mit einer Trenneinheit (21) von Wasser abgereichert und dann in Kolonne (4) oder auch direkt zugeführt werden.

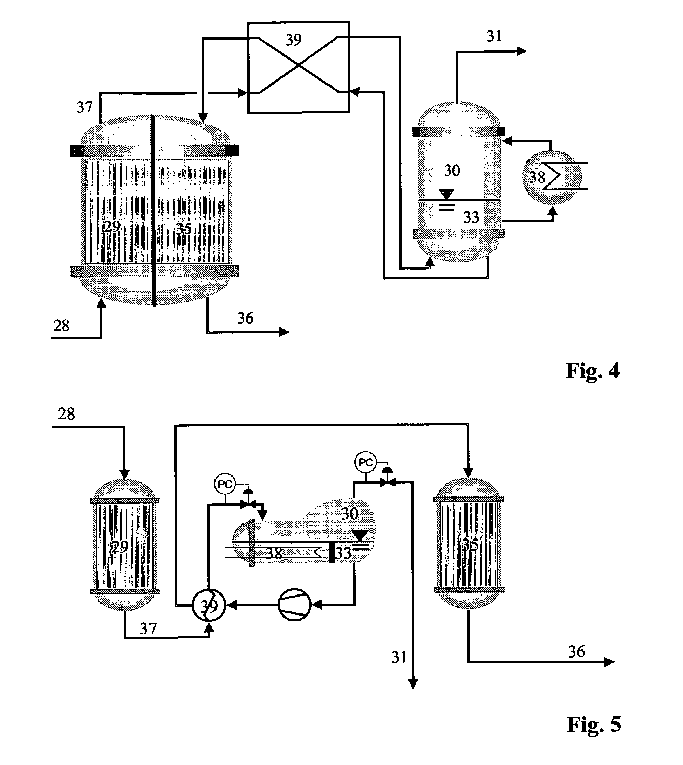

Einen anderen Aufbau eines solchen Reaktors zeigt Fig. 4. Der Reaktorzulauf (28) wird dem Reaktor wieder analog zu Fig. 3 von unten oder alternativ von oben zugeführt und in den ersten Reaktionsbereich (29) geleitet. Dort befindet sich der Katalysator in beheizbaren Rohren oder anderen beheizbaren Zwischenräumen. Durch die ablaufende Reaktion entsteht Isobuten und Wasser, bis maximal zum Erreichen des chemischen Gleichgewichts. Der Betriebsdruck des Reaktors wird ausreichend hoch gewählt, so dass keine Verdampfung im Reaktor auftritt. Der Reaktionsaustrag (37) wird in einen Behälter mit Verdampfer oder direkt in einen Verdampfer geführt und bildet durch die Wärmezufuhr mit dem Heizelement oder Verdampfer (38) eine Dampfphase (30), welche als Produktstrom (31) direkt in eine Kolonne zur Aufarbeitung, wie z. B. in Kolonne (4) der Fig. 1 oder 2 geleitet werden kann. Die flüssige Phase (33) kann mit einem Wärmeaustauscherelement (39) wieder auf die zulässige Reaktionstemperatur eingestellt werden und den Strom (37) vorwärmen, um auf diese Weise Energie einzusparen, bevor sie in den zweiten Reaktionsbereich (35), welcher ebenfalls wie der erste (29) mit Katalysator befüllt und beheizt werden kann, geleitet wird. Dort läuft die Reaktion wieder weiter bis maximal zum chemischen Gleichgewicht. Reaktionsbereich (29) und (35) sind baulich voneinander getrennt. Diese Bauart ist technisch einfach zu realisieren. Alternativ kann der Reaktor auch mit dem Reaktionsgemisch in beiden Reaktionsbereichen im Gleichstrom von unten nach oben oder von oben nach unten durchströmt werden. Die austretende Teilströme (36) und (31) entsprechen in Summe dem Strom (3) in Fig. 1 und 2. Während Strom (31) direkt in die Kolonne (4) eingeleitet wird, kann Strom (36) wie in Fig. 1 und 2 gezeigt erst mit einer Trenneinheit (21) von Wasser abgereichert und dann in Kolonne (4) oder auch direkt zugeführt werden.

Diese Verfahrensweise kann aber auch dadurch erreicht werden, dass das Verfahren in zumindest zwei Reaktoren durchgeführt wird, wobei der flüssige Reaktoraustrag aus dem ersten Reaktor in eine Zone überführt wird, in welcher ein Teil des Reaktoraustrags durch Änderung von Druck und/oder Temperatur in eine Gasphase überführt wird und von der flüssigen Phase abgetrennt wird und die flüssige Phase in den zweiten Reaktor überführt wird. Diese Zwischenverdampfung im zwei- oder mehrstufigen Reaktorsystem kann z. B. durch Verringerung des Drucks gegenüber dem Reaktionsdruck durchgeführt werden. Es kann vorteilhaft sein, wenn bei der Verdampfung im zwei- oder mehrstufigen Reaktorsystem ein Wärmeaustausch zwischen dem der Verdampfung zugeführten flüssigen Strom und dem von der Verdampfung rückgeführten flüssigen Strom durchgeführt wird. Auf diese Weise können Wärmeverluste vermieden werden.

Auf diese Weise müssen keine aufwändigen Apparaturen eingesetzt werden und die Apparatekosten des Reaktorsystems können niedrig gehalten werden. Eine entsprechende Verschaltung zeigt Fig. 5. Der Reaktorzulauf (28) wird analog zu Fig. 4 in den ersten Reaktionsbereich (29) geleitet. Dieser Reaktionsbereich besteht aus einem oder mehreren Reaktoren, in welchem/welchen sich der Katalysator in beheizbaren Rohren oder anderen beheizbaren Zwischenräumen befmdet. Der/die Reaktor(en) können von oben nach unten oder umgekehrt durchströmt werden. Durch die ablaufende Reaktion entsteht Isobuten und Wasser bis maximal zum Erreichen des chemischen Gleichgewichts. Der Betriebsdruck des Reaktors wird so hoch gewählt, dass keine Verdampfung im Reaktor auftritt. Der Reaktionsaustrag (37) wird in einen Behälter mit Verdampfer oder direkt in einen Verdampfer, bevorzugt einen Kettle-Verdampfer geführt und bildet durch die Wärmezufuhr mit dem Heizelement oder Verdampfer (38) eine Dampfphase (30), welcher als Produktstrom (31) direkt in eine Aufarbeitungskolonne, wie z. B. Kolonne (4) der Fig. 1 oder 2 geleitet werden kann. Das Verdampfen kann noch dadurch unterstützt werden, dass der Betriebsdruck des Verdampfers abgesenkt wird. Hierdurch wird das Produkt weniger stark überhitzt als in den oben aufgeführten Bauarten. Die flüssige Phase (33) kann mit einem Wärmeaustauscherelement (39) wieder auf die zulässige Reaktionstemperatur eingestellt werden und den Strom (37) vorzuwärmen, um derart Energie einzusparen, bevor sie in den zweiten Reaktionsbereich (35), welcher ebenfalls wie der erste (29) als einzelner oder mehrere Reaktoren ausgeführt wird und mit Katalysator befüllt und beheizt werden kann, geleitet wird. Dort läuft die Reaktion weiter bis maximal zum chemischen Gleichgewicht. Die austretende Teilströme (36) und (31) entsprechen in Summe dem Strom (3) in Fig. 1 und 2. Während Strom (31) direkt in die Kolonne (4) eingeleitet wird, kann Strom (36) wie in Fig. 1 und 2 gezeigt erst mit einer Trenneinheit (21) von Wasser abgereichert und dann in Kolonne (4) oder auch direkt zugeführt werden.

Der massenbezogene Dampfanteil, welcher in den Reaktoren gemäß Fig. 3 bis 5 erzeugt und als Strom (31) einer Aufarbeitungskolonne, wie z. B. Kolonne (4) der Fig. 1 oder 2 geleitet wird, beträgt vorzugsweise 1 bis 80 Massen-%, bevorzugt 5 bis 75 Massen-% des eingesetzten Stroms (28).

Mit dem erfindungsgemäßen Verfahren kann tert.-Butanol (TBA) in Isobuten und Wasser aufgespalten werden. Im erfindungsgemäßen Verfahren kann sowohl technischer, mit Oligomeren des Isobutens, beispielsweise Diisobuten, verunreinigter TBA als auch reiner TBA eingesetzt werden. Bevorzugt setzt man TBA/Wasser-Gemische ein, beispielsweise TBA/Wasser-Azeotrope, wie sie bei der destillativen Aufarbeitung oder direkt bei der TBA-Synthese anfallen.

Der eingesetzte TBA kann durch Umsetzung von Isobuten oder Isobuten-haltigen Kohlenwasserstoffgemischen, insbesondere Raffinat I oder selektivhydriertes Crack-C₄, mit Wasser gewonnen werden oder aus anderen technischen Prozessen stammen, bei denen TBA nicht unbedingt durch Addition hergestellt wurde, sondern als Neben- oder Koppelprodukt anfällt, wie beispielsweise bei der Synthese von tert.-Butylhydroperoxid oder bei Epoxidierungen mit tert.-Butylhydroperoxid.

Im nachfolgenden wird die weitere Ausgestaltung des erfindungsgemäßen Verfahrens am Beispiel der Aufspaltung von TBA beschrieben, ohne dass das erfindungsgemäße Verfahren auf diese Ausführungen beschränkt sein soll. Vielmehr ist es für den Fachmann ein leichtes die entsprechenden Verfahrensschritte für die Spaltung von anderen Additionsprodukten von Isoolefinen anzupassen.

Der TBA-Gehalt im Zulauf zum ersten Spaltungsreaktors, der frisches Edukt und TBA/Wasser-Rückführungsstrom aufweist, beträgt vorzugsweise von 40 bis 99 Massen-%, bevorzugt von 55 bis 98 Massen-%.

Die im Einzelfall besonders günstige Raumgeschwindigkeit hängt vom Wassergehalt des Reaktionsgemisches und in hohem Maße von der Reaktionstemperatur und von der Aktivität des verwendeten Katalysators ab und ist für jeden Katalysator individuell zu ermitteln.

Die Raumgeschwindigkeit (LHSV) in Liter Beschickung je Liter aufgequollener Katalysator je Stunde beträgt im Allgemeinem bei einer Reaktionstemperatur von 80 bis 100 °C von 10 bis 100 1/(1*h), bei einer Reaktionstemperatur von 100 bis 130 °C von 15 bis 300 1/(1*h) und bei einer Reaktionstemperatur von 130 bis 150 °C bis 600 1/(1*h). Doch es können auch geringere Raumgeschwindigkeiten gewählt werden.

Der Reaktoraustrag aus der erfindungsgemäßen Spaltung von TBA wird vorzugsweise in Isobuten, Nebenprodukte, Wasser und ein TBA/Wasser-Gemisch, das in den Reaktor zurückgefahren wird, mit einem Wassergehalt kleiner 20 Massen-%, vorzugsweise kleiner 18 Massen-%, bevorzugt kleiner 10 Massen-% und besonders bevorzugt mit einem Wassergehalt von 3 bis 9 Massen-%, getrennt.

Die Aufarbeitung des Reaktionsaustrages erfolgt vorzugsweise dadurch, dass aus dem Reaktoraustrag in einer ersten Kolonne über Kopf der überwiegende Teil des Isobutens als Isobuten/Wasser-Azeotrop abgetrennt wird. Aus dem Kopfprodukt der ersten Kolonne kann nach einer Abtrennung eines Teils des Wassers, z. B. durch Phasentrennung, durch azeotrope Destillation in einer zweiten Kolonne (16) ein nahezu wasserfreies Isobuten gewonnen werden. Mit dem erfindungsgemäßen Verfahren kann auf diese Weise ein Isobuten mit einer Reinheit größer 97, bevorzugt größer 98, besonders bevorzugt größer 99 und ganz besonders bevorzugt größer 99,9 Massen-% gewonnen werden, wobei der maximale Wassergehalt bei Werten kleiner 10000, bevorzugt kleiner 1000, besonders bevorzugt kleiner 250 und ganz besonders bevorzugt kleiner 50 ppm liegt. Ein Isobutenhaltiges Gemisch, welches Isobuten und Wasser in den oben genannten Konzentrationen aufwiest, ist ebenfalls Gegenstand der vorliegenden Erfindung.

C₈-Kohlenwasserstoffe und langkettigere Kohlenwasserstoffe, die als Nebenprodukte im Reaktionsaustrag vorliegen, können aus dem isobutenfreien Sumpfprodukt der ersten Kolonne (4) zum Beispiel in einer dritten Kolonne (6) als Kopfprodukt abgetrennt werden. Ebenso ist es aber möglich, die C₈-Kohlenwasserstoffe und eventuell langkettigere Kohlenwasserstoffe, die als Nebenprodukte im Reaktionsaustrag vorliegen, als Sumpfprodukt in einer vierten Kolonne (23), in der ein dampfförmiger oder flüssiger Seitenabzug der ersten Kolonne (4) destilliert wird, abzutrennen.

Es kann vorteilhaft sein, wenn aus dem Sumpfprodukt der ersten Kolonne (4) oder der dritten Kolonne (6) in einer fünften Kolonne (9) über Kopf ein Gemisch aus Wasser und TBA abgetrennt wird, welches gegebenenfalls Isobuten aufweisen kann und als Sumpfprodukt Wasser, welches gegebenenfalls organische Verunreinigungen aufweist, wobei das tert.-Butanol/Wasser-Gemisch zumindest teilweise in den Reaktor zur Spaltung des tert.-Butanols zurückgeführt wird. 2- Butanol, welches als Verunreinigung im Sumpfprodukt der ersten oder dritten Kolonne vorhanden sein kann, kann z. B. destillativ aus dem als Sumpfprodukt abgetrennten Wasser mittels einer weiteren Kolonne oder als Seitenstrom in der fünften Kolonne (9) abgetrennt werden.

Das Destillat (12) der Isobuten-Anreicherung (4a), welches im wesentlichen ein Isobuten/Wasser-Azeotrop aufweist, kann auf unterschiedliche Weise aufbereitet werden. Insbesondere kann es aber kondensiert und in einen Absitz- oder Trennbehälter überführt werden oder alternativ direkt in einen Absitz- oder Trennbehälter mit Wärmetauscherelementen kondensiert werden und einer Flüssig-Flüssig-Trennung unterzogen werden. Nach der Flüssig-Flüssig-Trennung kann ein Teil der organischen Flüssigkeit einer weiteren Kolonne (16) am Kopf oder im oberen Teil der Kolonne zugeführt werden und in welcher nahezu wasserfreies Isobuten (18) im Sumpf gewonnen wird und ein Isobuten/Wasser-Azeotrop über Kopf dieser Kolonne abgetrennt wird, welches wieder dem Flüssig-Flüssig-Trennbehälter zugeführt werden kann.

Zur Vermeidung von großen Prozessströmen kann es vorteilhaft sein, wenn dem Reaktionsaustrag vor dem Auftrennen in verschiedene Fraktionen in einer ersten Kolonne oder einer Teilfraktion nach dem Auftrennen ein Teil des im Reaktionsaustrag bzw. in den erhaltenen Fraktionen vorhandenen Wassers, z. B. durch Pervaporation und/oder Dampf-Permeation mittels einer Membran, abgetrennt wird. Vorzugsweise wird das gesamte im Reaktionsaustrag vorhandene Wasser bis auf den Teil, der gegebenenfalls für die destillative Abtrennung von Isobuten und C₈-Kohlenwasserstoffen durch Azeotropdestillation notwendig ist, abgetrennt. Eine Abtrennung eines Teils des Wassers aus dem Reaktionsaustrag vor dem Auftrennen in verschiedene Fraktionen in einer ersten Kolonne kann auch durch eine Phasenabscheidung in einem Trenn- oder Absitzbehälter erfolgen. Ebenso kann es vorteilhaft sein aus dem Reaktorzulauf vor dem Eintritt in den Reaktor, also aus Strom (1), Strom (10) und/oder dem daraus gemischten Strom, ein Teil des vorhandenen Wassers durch Pervaporation und/oder Dampfpermeation mittels einer Membran abzutrennen.

Die Abtrennung von Wasser aus diesen Strömen oder Fraktionen, die Gemische, die Wasser, TBA, Isobuten und C₈-Kohlenwasserstoffe und gegebenenfalls weitere organische Komponenten enthalten können, aufweisen, erfolgt an Membranen, die für Wasser, jedoch kaum oder gar nicht für die oben genannten organischen Stoffe durchlässig sind.

Die Abtrennung des Wassers mit Hilfe einer Membran kann durch Umkehr-Osmose (Retentat und Permeat sind flüssig), durch Pervaporation (flüssiges Retentat, dampfförmiges Permeat) oder durch Dampf-Permeation (Retentat und Permeat dampfförmig) erfolgen. Weiterhin ist eine Abtrennung durch gleichzeitige Pervaporation und Dampfpermeation möglich. Bevorzugt erfolgt die erfindungsgemäße Abtrennung des Wassers mit Hilfe einer Membran durch Pervaporation (flüssiges Retenat, dampfförmiges Permeat)

Zur Wasserabtrennung durch Umkehr-Osmose, Pervaporation oder Dampf-Permeation können handelsübliche hydrophile Membrane verwendet werden. Diese Membrane können Polymermembrane oder anorganische Membrane sein.

Im erfindungsgemäßen Verfahren können beispielsweise Polymermembrane der Firmen Sulzer Chemtech, CM-Celfa, GKSS oder Sophisticated Systems (Polyimidmembran) eingesetzt werden. Verwendbare Typen sind z. B. Pervap 2200, Pervap 2201, Pervap 2202 oder Pervap 2510 von Sulzer oder Typ 2S-DP-H018 von Sophisticated Systems. Als anorganische Membrane können beispielsweise die nachfolgend aufgeführten verwendet werden: SMS (Sulzer Chemtech); Silica (Pervatech); NaA (Mitsui oder Smart Chemical). Weiterhin können auch Kombinationen aus anorganischer Membran bzw. anorganischem Trägermaterial und einer Polymermembran bzw. aufgebrachten Polymertrennschicht eingesetzt werden.

Die erfindungsgemäße Wasserabtrennung erfolgt an den anorganischen Membranen bevorzugt bei einer Temperatur von 20 bis 200 °C und an den Polymermembranen bevorzugt bei einer Temperatur von 20 bis 150 °C. Besonders bevorzugt wird die Wasserabtrennung an beiden Membrantypen bei einer Temperatur von 60 bis 140 °C durchgeführt.

Das erfindungsgemäße Verfahren wird vorzugsweise bei einem Druck des der Membraneinheit zugeführten Gemisches (Flüssig, dampfförmig oder als Mischphase) von 0,5 bis 30 bar, bevorzugt von 0,8 bis 20 bar durchgeführt. Der Druck auf der Permeatseite der Membran beträgt vorzugsweise von 0,001 bis 1 bar.

Bei Polymermembranen beträgt der Differenzdruck vorzugsweise von 0,01 bis 20 bar und bei anorganischen Membranen vorzugsweise von 0,01 bis 30 bar. Besonders bevorzugt liegen die Differenzdrücke im Bereich von 1 bis 5 bar. Der Massenstrom (kg Permeat je Quadratmeter Membranoberfläche je Stunde) beträgt vorzugsweise von 0,1 bis 10 kg/(m²h), besonders bevorzugt von 1 bis 8 kg/(m²h). Das als Permeat abgetrennte Wasser weist vorzugsweise einen Gehalt an organischen Inhaltsstoffen, insbesondere an TBA von kleiner 10 Massen-%, bevorzugt kleiner 5 Massen-% und ganz besonders bevorzugt von 3 bis 0,05 Massen-% auf. Es können auch kleinere Werte bis zu 0,001 Massen-% erreicht werden, wobei dies meistens nicht erforderlich und wirtschaftlich auch nicht sinnvoll ist.

Die Wasserabtrennung durch Pervaporation kann wie bereits beschrieben an verschiedenen Stellen des Prozesses eingebunden sein. Geeignete Ströme sind beispielsweise das den Spaltreaktor verlassende Reaktionsgemisch oder das rückgeführte TBA/Wasser-Gemisch aus der Azeotropkolonne (Kolonne (9)). Das den Spaltreaktor verlassende Reaktionsgemisch ist, abgesehen von den Abwasserströmen, der Strom mit dem höchsten Wassergehalt. Daher lässt sich daraus im Vergleich zu anderen Strömen mit dem geringsten energetischen Aufwand ein relativ großer Teil des Wassers durch ein Membranverfahren abtrennen. Weitere Möglichkeiten sind im Reaktorzulauf zu finden. Dort kann zum Beispiel aus einem der Ströme (1), (10) und/oder dem daraus gemischten Strom, ein Teil des vorhandenen Wassers durch Pervaporation und/oder Dampfpermeation mittels einer Membran abgetrennt wird. Auf diese Weise lassen sich TBA-Gehalte größer als 90 Massen-% erreichen, welche einen besonders hohen Umsatz im Reaktor(system) ermöglichen.

Das Kopfprodukt (10) der Azeotropkolonne (9), das in den Reaktor (2) zurückgeführt wird, hat etwa eine Azeotrop-nahe Zusammensetzung, also einen Wassergehalt von ca. 20 bis 12 Massen-%. Durch Einsatz eines Membranverfahrens kann der Wassergehalt darin auf Werte kleiner 12 Massen-%, insbesondere 1 bis 10 Massen-%, gesenkt werden, sodass besonders wenig Wasser in den Reaktor zurückgeführt wird, wodurch die Raum-Zeit-Ausbeute im Spaltreaktor ansteigt. In einer anderen Variante der Verschaltung in Fig. 2 kann das Membranverfahren (21) genutzt werden, um den Wasserhaltigen Strom (26), der als Sumpf der ersten Trennkolonne (4) anfällt und der teilweise in den Reaktor zurückgefahren wird, zu entwässern.

Natürlich ist es auch möglich, Wasser an mehreren Stellen des Verfahrens mittels Membranen, z. B. an beiden der oben genannten Stellen, abzutrennen.

Wird Wasser mit Hilfe einer Membran abgetrennt, sind zwei Grundvarianten möglich:
a) Das gesamte Reaktionswasser wird gegebenenfalls bis auf einen kleinen Rest, der für die Abtrennung von C₈-Kohlenwasserstoffen und Isobuten durch Azeotropdestillation notwendig ist, abgetrennt.
b) Es wird nur ein Teil des Reaktionswasser durch ein Membranverfahren entfernt.
Im Fall (a) kann auf die TBA/Wasser-Azeotropkolonne verzichtet werden.

Ein Blockschema einer Anlage (Verschaltung), in der das erfindungsgemäße Verfahren durchgeführt werden kann, ist in Figur 1 dargestellt. Das Einsatzstoff(gemisch) (1) wird zusammen mit dem TBA/Wasser-Azeotrop, bzw. mit Azeotrop-naher Zusammensetzung (10) in den Reaktor (2) eingespeist. Der Reaktor (2) kann hierbei aus einem einzelnen Reaktor oder einem Reaktorsystem bestehen (s.u.). Das den Reaktor (2) verlassende Reaktionsgemisch (3) wird in die Kolonne (4) eingeleitet. Optional kann das den Reaktor (2) verlassende Reaktionsgemisch (3) entwässert und somit weitgehend von Reaktionswasser befreit werden, bevor es in die Kolonne (4) eingeleitet wird. Als Entwässerungsmodul (21) kann im einfachsten Fall ein Dekanter (Abscheide- oder auch Absitzbehälter) eingesetzt werden, welcher ein eventuell entstehendes zweiphasiges Gemisch in einen organischen Flüssigkeitsstrom (3A) und einen wässrigen Flüssigkeitsstrom (3B) trennt. Deutlich mehr Wasser lässt sich mit Hilfe eines Membranverfahrens abtrennen, welches ebenfalls einen organischen Flüssigkeitsstrom (3A) und einen wässrigen Flüssigkeitsstrom (3B) erzeugt. Der organische Flüssigkeitsstrom (3A) wird in die Kolonne (4) eingeleitet und der wässrige Flüssigkeitsstrom (3B) in Kolonne (9) zum Ausschleusen und weiteren Aufreinigen des Wassers oder der direkten Entsorgung. Als Kopfprodukt (12) der Kolonne (4) (4a + 4b) wird Isobuten mit geringen Mengen Wasser abgetrennt, das im Absitzbehälter (13) in eine Isobuten- und eine Wasserphase getrennt wird. Die Wasserphase (19) kann in die Kolonne (9) gefahren werden. Ein Teil der Isobutenphase wird als Rücklaufstrom auf den Kopf der Kolonne (4) gefahren, der andere Teil (15) wird in Kolonne (16) in Rein-Isobuten (18) und in ein Wasser/Isobuten-Azeotrop (17), das in den Trennbehälter (13) zurückgeleitet wird, aufgetrennt. Um die Abtrennung des TBA vom Isobuten in Kolonne (4) zu verbessern, kann optional ein Frischwasserstrom (20) am Kopf oder im Verstärkungsteil der Kolonne (4) zugegeben werden. Vom Sumpfprodukt (5) der Kolonne (4) werden in Kolonne (6) C₈-Kohlenwasserstoffe als Kopfprodukt (7) abgezogen. Das Sumpfprodukt (8) wird in Kolonne (9) in Wasser mit eventuell vorhandenen organischen Komponenten (wie z. B. 2-Butanol und/oder C₁₂-Kohlenwasserstoffe) (11) und ein TBA/Wasser-Azeotrop (10), das gegebenenfalls geringe Mengen Isobuten enthalten kann (aus Strom (19)), getrennt. Strom (10) wird in den Reaktor (2) zurückgefahren. Eventuell vorhandene organischen Komponenten (wie z. B. 2-Butanol (SBA) und/oder C₁₂-Kohlenwasserstoffe) im Abwasserstrom (11) können durch Destillation leicht von reinem Wasser getrennt werden. Alternativ kann 2-Butanol gezielt ausgeschleust werden, indem ein Strom aus der Dampfphase des Verdampfers der Kolonne oder dampfförmig oder flüssig als Seitenstrom (11A) im Abtriebsteil der Kolonne (9) abgezogen wird.

Ein Blockschema einer alternativen Anlage, in der das erfindungsgemäße Verfahren durchgeführt werden kann, ist in Figur 2 dargestellt. Der(Das) Einsatzstoff(gemisch) (1) wird zusammen mit dem TBA/Wasser-Azeotrop, bzw. mit Azeotrop-naher Zusammensetzung (10) in den Reaktor (2) eingespeist. Der Reaktor (2) kann hierbei aus einem einzelnen oder einem Reaktorsystem bestehen (s. u.). Das den Reaktor (2) verlassende Reaktionsgemisch (3) wird in die Kolonne (4) eingeleitet. Optional kann das den Reaktor (2) verlassende Reaktionsgemisch (3) entwässert und somit weitgehend von Reaktionswasser befreit werden bevor es in die Kolonne (4) eingeleitet wird. Als Entwässerungsmodul (21) kann im einfachsten Fall ein Dekanter (Abscheide- oder auch Absitzbehälter) eingesetzt werden, welcher eventuell entstehendes zweiphasiges Gemisch in eine organischen Flüssigkeitsstrom (3A) und einen wässrigen Flüssigkeitsstrom (3B) trennt. Deutlich mehr Wasser lässt sich mit Hilfe eines Membranverfahrens abtrennen, welches ebenfalls einen organischen Flüssigkeitsstrom (3A) und einen wässrigen Flüssigkeitsstrom (3B) erzeugt. Der organische Flüssigkeitsstrom (3A) wird in die Kolonne (4) eingeleitet und der wässrige Flüssigkeitsstrom (3B) in Kolonne (9) zum Ausschleusen und weiteren Aufreinigen des Wassers oder der direkten Entsorgung. Als Kopfprodukt (12) der Kolonne (4) wird Isobuten mit geringen Mengen Wasser abgetrennt, das im Absitzbehälter (13) in eine Isobuten- und eine Wasserphase getrennt wird. Die Wasserphase (19) kann in die Kolonne (9) gefahren werden. Ein Teil der Isobutenphase wird als Rücklaufstrom auf den Kopf der Kolonne (4) gefahren, der andere Teil (15) wird in Kolonne (16) in Rein-Isobuten (18) und in ein Wasser/Isobuten-Azeotrop (17), das in den Trennbehälter (13) zurückgeleitet wird, aufgetrennt. Um die Abtrennung des TBA vom Isobuten in Kolonne (4) zu verbessern, kann optional ein Frischwasserstrom (20) am Kopf oder im Verstärkungsteil der Kolonne (4) zugegeben werden. Das Sumpfprodukt (5) wird in Kolonne (9) in Wasser mit eventuell vorhandenen organischen Komponenten (wie z. B. 2-Butanol und/oder C₁₂-Kohlenwasserstoffe) (11) und ein TBA/Wasser-Azeotrop (10), das gegebenenfalls geringe Mengen Isobuten enthalten kann (aus Strom (19)), getrennt. Strom (10) wird in den Reaktor (2) zurückgefahren. Die Brüden (27) der Kolonne (9) können ganz oder teilweise zur Energieeinsparung und Konzentrationseinstellung in Kolonne (4) eingeleitet werden. Ein Teil (26) des Sumpfstroms (5) kann direkt in den Reaktor zugeführt werden. Eventuell vorhandenen organischen Komponenten (wie z. B. 2-Butanol und/oder C₁₂-Kohlenwasserstoffe) im Abwasserstrom (11) können durch Destillation leicht von reinem Wasser getrennt werden. Um C₈-Kohlenwasserstoffe abzutrennen, wird ein dampfförmiger oder flüssiger Seitenstrom (22) der Kolonne (4) entnommen und in Kolonne (23) als Sumpfprodukt (24) mit TBA und Wasser abgezogen. Als Kopfprodukt (25) wird Isobuten und TBA zurückgewonnen und in die Kolonne (4) zurückgeführt.

Als weitere Option besteht die Möglichkeit den Zulaufstrom (1) zunächst in einen oder mehrere weitere Reaktor(en) zu leiten und das vorreagierte Gemisch in Kolonne (4a) einzuspeisen.

Die destillative Auftrennung von bei dem erfindungsgemäßen Verfahren anfallenden Stoffströmen, kann in ein oder mehreren Kolonnen mit Einbauten, die aus Böden, rotierenden Einbauten, ungeordneten und/oder geordneten Schüttungen oder Packungen bestehen, durchgeführt werden. Vorzugsweise erfolgt die destillative Abtrennung des Isobuten aus dem Reaktoraustrag in einer einzigen Kolonne.

Bei den Kolonneneinbauten können z. B. folgende Typen zum Einsatz kommen:
Böden mit Bohrungen oder Schlitzen in der Bodenplatte.
Böden mit Hälsen oder Kaminen, die von Glocken, Kappen oder Hauben überdeckt sind.
Böden mit Bohrungen in der Bodenplatte, die von beweglichen Ventilen überdeckt sind.
Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern eingesetzt werden. Sie können aus fast allen Werkstoffen, wie z. B. Stahl, Edelstahl, Kupfer, Kohlenstoff, Steingut, Porzellan, Glas, Kunststoffen usw. oder Mischungen davon bestehen und in verschiedenen Formen, wie z. B. Kugeln, Ringen mit glatten oder profilierten Oberflächen, Ringen mit Innenstegen oder Wanddurchbrüchen, Drahtnetzringen, Sattelkörper oder Spiralen vorliegen.

Packungen mit regelmäßiger Geometrie können z. B. aus Blechen oder Geweben aus Metall oder Kunststoff bestehen. Beispiele solcher Packungen sind Sulzer Gewebepackungen BX, Sulzer Lamellenpackungen Mellapak aus Metallblech, Hochleistungspackungen wie MellapakPlus, Strukturpackungen von Sulzer (Optiflow), Montz (BSH) und Kühni (Rombopak).

Der Betriebsdruck der im erfindungsgemäßen Verfahren eingesetzten Kolonnen, insbesondere der Kolonnen (4), (6), (9), (16) und (23) beträgt vorzugsweise von 0,5 bis 15 bar_{abs.} (bara), besonders bevorzugt von 1 bis 10 bara.

Die zur Trennung des Reaktoraustrags eingesetzte Kolonne (4) weist vorzugsweise eine theoretische Trennstufenzahl von 5 bis 80, bevorzugt eine theoretische Trennstufenzahl von 10 bis 60 auf. Der Zulaufboden hängt von der Zusammensetzung des Zulaufs ab. Es hat sich als vorteilhaft erwiesen, wenn die Einspeisung des Reaktoraustrags auf den, von oben gezählten, 4. bis 55. theoretischen Boden, insbesondere auf den 5. bis 50. im unteren Abschnitt der Kolonne, insbesondere der Kolonne (4a) der Fig. 1 und Fig. 2 erfolgt. Die Zufuhr des Frischwassers (21) erfolgt am Kopf der Kolonne (4) auf Stufe 1. Ebenso der Rücklauf (14). Die Zufuhr des optionalen Dampfstroms (27) der Kolonne (9) erfolgt im Sumpf der Kolonne (4).

In einer besonderen Ausführungsart (Verschaltung) der vorliegenden Erfindung gemäß Fig. 2 hat es sich als vorteilhaft erwiesen, wenn die dampfförmige oder flüssige Entnahme des Stroms (22) auf der, von oben gezählten, 4. bis 65. theoretischen Stufe, insbesondere auf den 5. bis 60. der Kolonne (4) der Fig. 2 erfolgt. Die Rückspeisung des isobutenreichen Stroms (25) erfolgt an gleicher oder bevorzugt höherer Stelle (weiter oben in der Kolonne (4a), d. h. auf der, von oben gezählten, 2. bis 65. theoretischen Stufe, insbesondere auf der 3. bis 60. der Kolonne (4) der Fig. 2.

Die zur Ausschleusung des Abwassers und Aufkonzentrierung von TBA eingesetzte Kolonne (9) weist vorzugsweise eine theoretische Trennstufenzahl von 5 bis 70, bevorzugt eine theoretische Trennstufenzahl von 8 bis 65 auf. Der Zulaufboden hängt von der Zusammensetzung des Zulaufs ab. Es hat sich als vorteilhaft erwiesen, wenn die Einspeisung des Stroms (5) in Fig. 2, bzw. des Stroms (8) in Fig. 1 auf den, von oben gezählten, 1. bis 55. theoretischen Boden, insbesondere auf den 1. bis 45. erfolgt. Die Zufuhr des möglichen Abwassers (3B) und (19) erfolgt auf den, von oben gezählten, 1. bis 60. theoretischen Boden, insbesondere auf den 1. bis 50. Boden.

Die zur Trocknung des Isobutens eingesetzte Kolonne (16) weist vorzugsweise eine theoretische Trennstufenzahl von 4 bis 35, bevorzugt eine theoretische Trennstufenzahl von 5 bis 30 auf. Es hat sich als vorteilhaft erwiesen, wenn die Einspeisung des Zulaufs (15) auf den, von oben gezählten, 1. bis 6. theoretischen Boden, insbesondere auf den 1. bis 4. erfolgt.

Die in Fig. 1 zur Ausschleusung von C₈-Kohlenwasserstoffen eingesetzte Kolonne (6) weist vorzugsweise eine theoretische Trennstufenzahl von 4 bis 75, bevorzugt eine theoretische Trennstufenzahl von 5 bis 50 auf. Der Zulaufboden hängt von der Zusammensetzung des Zulaufs ab. Es hat sich als vorteilhaft erwiesen, wenn die Einspeisung des Stroms (5) in Fig. 1 auf den, von oben gezählten, 1. bis 55. theoretischen Boden, insbesondere auf den 1. bis 45. erfolgt.

Die in Fig. 2 zur Ausschleusung von C₈-Kohlenwasserstoffen eingesetzte Kolonne (23) weist vorzugsweise eine theoretische Trennstufenzahl von 4 bis 65, bevorzugt eine theoretische Trennstufenzahl von 5 bis 50 auf. Es hat sich als vorteilhaft erwiesen, wenn die Einspeisung des Zulaufs (22) auf den, von oben gezählten, 1. bis 25. theoretischen Boden, insbesondere auf den 1. bis 15. erfolgt. Der Produktstrom (25) wird dampfförmig oder flüssig als Destillat, bevorzugt dampfförmig am Kopf der Kolonne abgezogen.

In den Blockschaltbildern sind gewöhnliche Bauteile wie Pumpen, Verdichter, Ventile, Wärmetauscher und Verdampfer nicht dargestellt, jedoch sind diese selbstverständliche Bauteile einer Anlage.

Das Reaktorsystem zur isothermen Durchführung von Gleichgewichtsreaktionen in der Flüssigphase an Festbettkatalysatoren, bei denen zumindest eines der Reaktionsprodukte unter Temperatur und Druckbedingungen, bei denen alle Edukte sowie zumindest ein Reaktionsprodukt überwiegend flüssig vorliegen, zum Teil, vorzugsweise zum überwiegenden Teil gasförmig vorliegt, insbesondere zur Durchführung des erfindungsgemäßen Verfahren zeichnet sich insbesondere dadurch aus, dass das Reaktorsystem zumindest zwei baulich voneinander getrennte Reaktionszonen aufweist, die jeweils einen im Festbett angeordneten Katalysator sowie Vorrichtungen zur isothermen Temperaturführung aufweisen, wobei zwischen zwei Reaktionszonen jeweils eine Abtrennzone vorhanden ist, die Mittel aufweist, mit denen zumindest ein Teil zumindest eines der Produkte der Gleichgewichtsreaktion durch Druck oder Temperaturänderung in die Gasphase überführt, vom Reaktionsgemisch abgetrennt und aus dem Reaktor ausgeschleust wird und mit denen das restliche Reaktionsgemisch in die folgende Reaktionszone überführt wird, wobei gegebenenfalls ein weiteres Mittel vorhanden sein kann, mit welchem die Temperatur oder der Druck in der folgenden Reaktionszone genau so eingestellt wird wie in der vorangegangenen Reaktionszone. Die Mittel zur Einstellung der Temperatur können z. B. übliche Platten- oder Rohr-Wärmetauscher sein. Das Mittel zur Überführung zumindest eines der Produkte der Gleichgewichtsreaktion in die Gasphase kann ebenfalls ein Wärmetauscher sein, so dass durch Eintrag von Wärmeenergie in das Reaktionsgemisch dieses Reaktionsprodukt verdampft wird. Ebenso ist es möglich, dass das Mittel ein Kettle-Verdampfer oder ein Behälter mit Beheizung ist, mit welchem eine Druckabnahme beim Eintritt der Reaktionsmischung in die Abtrennzone erzielt wird. Bei einer solchen Ausführungsart des Reaktors wird dieser in der auf die Abtrennzone folgenden Reaktionszone mit einem niedrigeren Druck gefahren als in der vorangegangenen Reaktionszone.

Die folgenden Beispiele sollen die Erfindung erläutern.

### 1. Beispiel

Die Herstellung von Isobuten erfolgte in einer nach Figur 1 und Fig. 5 realisierten Anlage, mit der Besonderheit, dass keine Wasserabtrennung mit dem Modul (21) erfolgte, und damit die Ströme 3 und 3A die gleiche Zusammensetzung hatten. Der Frisch-Wasserstrom (20) und der Abwasserstrom (3B) entfielen. Der Abwasserstrom (19) wurde direkt entsorgt.

Der Reaktionsteil (2) wurde zweistufig nach Fig. 5 realisiert. Der Reaktor (29) bestand aus einem, bei 120 °C isotherm betriebenen Labor-Rohrreaktor mit einem Katalysatorvolumen von ca. 310 ml. Als Katalysator wurde Amberlyst 15 eingesetzt. Der zweite Reaktor (35) bestand ebenfalls aus einem, bei 120 °C isotherm betriebenen Labor-Rohrreaktor mit einem Katalysatorvolumen von ca. 310 ml. Als Katalysator wurde ebenfalls Amberlyst 15 eingesetzt. Zur Zwischenverdampfung wurde ein beheizbarer Behälter eingesetzt.

Der Kolonnendurchmesser der Kolonnen (4), (6), (9) und (16) betrug jeweils 50 mm. Es wurden Metall-Füllkörper verwendet. In Kolonne (4) wurden ca. 30 theoretischen Stufen installiert, der Zulauf des Reaktoraustrags (3) erfolgte auf der, von oben gezählten, 20. theoretischen Stufe. Der Rücklauf (14) wurde am Kopf der Kolonne auf Stufe 1 zugegeben. In Kolonne (6) wurden ca. 18 theoretischen Stufen installiert, der Zulauf (5) aus Kolonne (4) erfolgte auf der, von oben gezählten, 12. theoretischen Stufe. In Kolonne (9) wurden ca. 13 theoretischen Stufen installiert, der Zulauf (8) aus Kolonne (6) erfolgte auf der, von oben gezählten, 2. theoretischen Stufe. Der Seitenstrom (11A) wurde dampfförmig auf der, von oben gezählten, ca. 10. theoretischen Stufe entnommen.

Der Zulauf (1), der für die Versuche verwendet wurde, wurde aus einer großtechnischen Anlage zur Herstellung von Isobuten entnommen und wies die in Tabelle 1 angegebene Zusammensetzung auf. C₈-Kohlenwasserstoffe und andere Hochsieder sind zum Teil bereits in geringen Mengen im Zulauf enthalten und werden zum Teil bei der Spaltung des TBA im Reaktorsystem gebildet. Die Stromnummern in der folgenden Tabelle entsprechen den Bezeichnungen in Fig. 1, im Reaktionsteil entsprechen sie Fig. 5. Komponenten mit einer Konzentration kleiner als 0,1 Massen-Teile im Gemisch sind in der Regel nicht in der Tabelle aufgeführt.

**Tabelle 1**

| Stromnummer | Stombezeichnung | Massenfluß [kg/h] | Konzentration der abzutrennenden Komponente in Massenteile |
|---|---|---|---|
| 1 | Frisch-Zulauf | 2,5 | 9,4 Wasser |
| | | | 90,5 TBA |
| | | | 0,1 SBA (2-Butanol) |
| 3 | Reaktorgesamtaustrag | 7,891 | 21,4Isobuten |
| | | | 21,4 Wasser |
| | | | 57,0 TBA |
| | | | 0,2 SBA |
| 5 | Sumpf der Kolonne (4) | 6,187 | 27,1 Wasser |
| | | | 72,7 TBA |
| | | | 0,2 SBA |
| 7 | Destillat der Kolonne (6) | 0,003 | 13,0 Wasser |
| | | | 58,2 TBA |
| | | | 28,8 C₈ |
| 8 | Sumpf der Kolonne (6) | 6,185 | 27,1 Wasser |
| | | | 72,7 TBA |
| | | | 0,2 SBA |
| 10 | Destillat der Kolonne (9) | 5,391 | 17,0 Wasser |
| | | | 82,8 TBA |
| | | | 0,2 SBA |
| 11A | Seitenstromentnahme der Kolonne (9) | 0,04 | 21,3 Wasser |
| | | | 74,9 TBA |
| | | | 3,7 SBA |
| | | | 0,1 sonstige Stoffe |
| 11 | Sumpf der Kolonne (9) | 0,754 | 99,995 Wasser |
| | | | 0,005 sonstige Stoffe |
| 12 | Destillat der Kolonne (4) | 2,503 | 99,3 Isobuten |
| | | | 0,6 Wasser |
| 14 | Externer Rücklauf zur Kolonne (4) | 0,8 | 99,9 Isobuten |
| | | | 0,1 Wasser |
| 15 | Zulauf zur Kolonne (16) | 1,986 | 99,9 Isobuten |
| | | | 0,1 Wasser |
| 17 | Destillat der Kolonne (16) | 0,297 | 99,5 Isobuten |
| | | | 0,5 Wasser |
| 18 | Sumpf der Kolonne (16) | 1,689 | 99,96 Isobuten |
| | | | 0,04 sonstige Stoffe |
| 19 | Abwasser aus Trennbehälter (13) | 0,015 | 99,9 Wasser |
| | | | 0,1 TBA |
| 28 | Zulauf zum Reaktor (29) | 7,891 | 14,6 Wasser |
| | | | 85,2 TBA |
| | | | 0,2 SBA |
| 31 | Abgetrennte Dampfphase (30) | 1,13 | 61,5 Isobuten |
| | | | 8,5 Wasser |
| | | | 30,0 TBA |
| | | | 0,1 SBA |
| 36 | Austrag des Reaktors (35) | 6,76 | 14,7Isobuten |
| | | | 23,6 Wasser |
| | | | 61,5 TBA |
| | | | 0,2 SBA |
| 37 | Austrag des Reaktors (29) | 7,891 | 17,3Isobuten |
| | | | 20,1 Wasser |
| | | | 62,4 TBA |
| | | | 0,2 SBA |

Der Druck des Reaktors (29) betrug 19 bar_{abs.,} der Druck des Reaktors (35) betrug 18 bar_{abs.,} und die Zwischenverdampfung (38) wurde bei 15 bar_{abs.} durchgeführt. Die Kolonnen (4), (6), (9) und (16) wurden bei ca. 6 bar_{abs.} Kopfdruck betrieben. Kolonne (4) wurde mit einem Dephlegmator ausgerüstet und mit einem Rücklaufstrom von ca. 11,5 kg/h betrieben. Es ergab sich eine Raumzeitausbeute von 2,7 kg/(1*h) bezogen auf die gesamte eingesetzte Katalysatormenge.

### 2. Beispiel

Die Herstellung von Isobuten erfolgte in einer nach Figur 1 realisierten Anlage, mit der Besonderheit, dass keine Wasserabtrennung mit dem Modul (21) erfolgte, und damit die Ströme 3 und 3A die gleiche Zusammensetzung hatten. Der Frisch-Wasserstrom (20) und der Abwasserstrom (3B) entfielen. Der Abwasserstrom (19) wurde direkt entsorgt.

Der Reaktionsteil (2) wurde einstufig realisiert. Der Reaktor (2) bestand aus einem, bei 120 °C isotherm betriebenen Labor-Rohrreaktor mit einem Katalysatorvolumen von ca. 285 ml. Als Katalysator wurde Amberlyst 15 eingesetzt. Der Kolonnendurchmesser der Kolonnen (4), (6), (9) und (16) betrug dabei jeweils 50 mm. Es wurden jeweils Metall-Füllkörper (Pallringe) verwendet. In Kolonne (4) wurden ca. 30 theoretischen Stufen installiert, der Zulauf des Reaktoraustrags (3) erfolgte auf der, von oben gezählten, 20. theoretischen Stufe. Der Rücklauf (14) wurde am Kopf der Kolonne auf Stufe 1 zugegeben. In Kolonne (6) wurden ca. 18 theoretischen Stufen installiert, der Zulauf (5) aus Kolonne (4) erfolgte auf der, von oben gezählten, 12. theoretischen Stufe. In Kolonne (9) wurden ca. 13 theoretischen Stufen installiert, der Zulauf (8) aus Kolonne (6) erfolgte auf der, von oben gezählten, 2. theoretischen Stufe. Der Seitenstrom (11A) wurde dampfförmig auf der, von oben gezählten, ca. 10. theoretischen Stufe entnommen.

Der Zulauf (1), der für die Versuche verwendet wurde, wurde aus einer großtechnischen Anlage zur Herstellung von Isobuten entnommen und wies die in Tabelle 2 angegebene Zusammensetzung auf. C₈-Kohlenwasserstoffe und andere Hochsieder sind zum Teil bereits in geringen Mengen im Zulauf enthalten und werden zum Teil bei der Spaltung des TBAs im Reaktorsystem gebildet. Die Stromnummern in der folgenden Tabelle entsprechen den Bezeichnungen in Fig. 1. Komponenten mit einer Konzentration kleiner als 0,1 Massen-Teile im Gemisch sind in der Regel nicht in der Tabelle aufgeführt.

**Tabelle 2**

| Stromnummer | Strombezeichnung | Massenfluss [kg/h] | Konzentration der abzutrennenden Komponente in Massenteile |
|---|---|---|---|
| 1 | Frisch-Zulauf | 2,5 | 9,4 Wasser |
| | | | 90,5 TBA |
| | | | 0,1 SBA |
| 3 | Reaktoraustrag | 9,756 | 17,3Isobuten |
| | | | 20,1 Wasser |
| | | | 62,4 TBA |
| | | | 0,2 SBA |
| 5 | Sumpf der Kolonne (4) | 7,988 | 23,6 Wasser |
| | | | 76,2 TBA |
| | | | 0,2 SBA |
| 7 | Destillat der Kolonne (6) | 0,004 | 3,3 Isobuten |
| | | | 12,5 Wasser |
| | | | 65,2 TBA |
| | | | 19,1 C₈ |
| 8 | Sumpf der Kolonne (6) | 7,984 | 23,6 Wasser |
| | | | 76,2 TBA |
| | | | 0,2 SBA |
| 10 | Destillat der Kolonne (9) | 7,256 | 16,4 Wasser |
| | | | 83,4 TBA |
| | | | 0,2 SBA |
| 11A | Seitenstromentnahme der Kolonne (9) | 0,040 | 22,7 Wasser |
| | | | 73,6 TBA |
| | | | 3,7 SBA |
| | | | 0,1 sonstige Stoffe |
| 11 | Sumpf der Kolonne (9) | 0,688 | 99,995 Wasser |
| | | | 0,005 sonstige Stoffe |
| 12 | Destillat der Kolonne (4) | 2,568 | 96,8 Isobuten |
| | | | 3,2 Wasser |
| 14 | Externer Rücklauf zur Kolonne (4) | 0,800 | 99,9 Isobuten |
| | | | 0,1 Wasser |
| 15 | Zulauf zur Kolonne (16) | 1,988 | 99,9 Isobuten |
| | | | 0,1 Wasser |
| 17 | Destillat der Kolonne (16) | 0,300 | 99,5 Isobuten |
| | | | 0,5 Wasser |
| 18 | Sumpf der Kolonne (16) | 1,688 | 99,965 Isobuten |
| | | | 0,045 sonstige Stoffe |
| 19 | Abwasser aus Trennbehälter (13) | 0,081 | 99,9 Wasser |
| | | | 0,1 TBA |

Der Druck des Reaktors (2) betrug 19 bar_{abs.}. Die Kolonnen (4), (6), (9) und (16) wurden bei ca. 6 bar_{abs.} Kopfdruck betrieben. Kolonne (4) wurde mit einem Dephlegmator ausgerüstet und mit einem Rücklaufstrom von ca. 5,1 kg/h betrieben. Es ergab sich eine Raumzeitausbeute von 5,8 kg/(1*h) bezogen auf die Gesamtmenge an eingesetztem Katalysator.

Die Bespiele zeigen eindrucksvoll, wie mit der neuen Anlage zur Herstellung von Isobuten die Kreislaufströme durch den Reaktor verringert werden können: In DE 3151446 werden Beispiele aufgeführt, in welchen das Verhältnis der Massenströme von Frisch-Zulauf zu Kreislaufströmen mit Werten von 1:7,4 bis 1:16,6 angegeben ist (siehe dort: Tabelle 1), während Beispiel 2 ein Verhältnis der Massenströme von Frisch-Zulauf (1) zum Kreislaufstrom (10) von 1:2,9 erreicht und in Beispiel 1 durch den höheren Teilumsatz im Reaktorsystem ein Verhältnis von 1:2,16 erreicht. Diese Werte können noch weiter verbessert werden, indem zum Beispiel in Kolonne (9) die Aufkonzentrierung zum TBA/Wasser-Azeotrop noch stärker durchgeführt wird.

## Patentansprüche

1. Verfahren zur Herstellung von Isobuten durch Spaltung von tert.-Butanol in zumindest einem Reaktor an einem in diesem Reaktor im Festbett angeordneten stark saueren Ionenaustauscherharz zu Isobuten und Wasser bei einer Temperatur von 80 bis 150 °C und einem Druck von 5 bis 25 bar und anschließende Auftrennung des Reaktionsgemisches in Isobuten, Nebenprodukte, Wasser und zumindest eine Mischung aus nicht gespaltenem tert.-Butanol und Wasser,
**dadurch gekennzeichnet,**
**dass** der Reaktor quasi isotherm mit einer Temperaturdifferenz zwischen den ein- und austretenden Strömen von weniger als 15 K betrieben wird, und das Verfahren so durchgeführt wird, dass die Spaltung in der ersten Reaktionszone bis zum Gleichgewicht der Spaltungsreaktion durchgeführt wird, in einer anschließenden Abtrennzone Isobuten gasförmig abgetrennt wird und in einer sich daran anschließenden Reaktionszone die Spaltung wieder bis zum Gleichgewicht der Spaltungsreaktion durchgeführt wird, und, wenn weitere Abtrennzonen und Reaktionszonen vorhanden sind, diese Verfahrensschritte entsprechend häufig wiederholt werden.

2. Verfahren nach Anspruch 1,
**dadurch gekennzeichnet,**
**dass** der Zulauf zum Reaktor sich aus frischem Eduktstrom und einem oder mehreren Rückführungsströmen, die eine Mischung aus nicht gespaltenem tert.-Butanol, Wasser und gegebenenfalls Nebenprodukten aufweist, zusammensetzt, wobei das Massen-Verhältnis von frischem Eduktstrom zu der Summe der Rückführungsströme kleiner 1 : 10 beträgt.

3. Verfahren nach Anspruch 1 oder 2,
**dadurch gekennzeichnet,**
**dass** mehrere Reaktoren, die in Reihe oder parallel angeordnet sind, eingesetzt werden, wobei alle Reaktoren isotherm betrieben werden.

4. Verfahren nach einem der Ansprüche 1 bis 3,
**dadurch gekennzeichnet,**
**dass** zumindest ein Reaktor eingesetzt wird, der zumindest zwei Reaktionszonen aufweist, die jeweils ein im Festbett angeordneten stark saueres Ionenaustauscherharz aufweisen, wobei zwischen zwei Reaktionszonen jeweils eine Abtrennzone vorhanden ist, in der zumindest ein Teil eines der Produkte der Spaltung durch Überführung in die Gasphase vom Reaktionsgemisch abgetrennt wird und aus dem Reaktor ausgeschleust wird.

5. Verfahren nach einem der Ansprüche 1 bis 4,
**dadurch gekennzeichnet,**
**dass** der Druck und die Temperatur in den Reaktionszonen des Reaktors so gewählt werden, dass das Reaktionsgemisch in flüssiger Phase vorliegt, und der Druck und die Temperatur in den Abtrennzonen so gewählt werden, dass das Isobuten zum Teil in der Gasphase vorliegt.

6. Verfahren nach einem der Ansprüche 1 bis 5,
**dadurch gekennzeichnet,**
**dass** das Verfahren in zumindest zwei Reaktoren durchgeführt wird, wobei der flüssige Reaktoraustrag aus dem ersten Reaktor in eine Zone überführt wird, in welcher ein Teil des Reaktoraustrags durch Änderung von Druck und/oder Temperatur in eine Gasphase überführt wird und von der flüssigen Phase abgetrennt wird und die flüssige Phase in den zweiten Reaktor überführt wird.

7. Verfahren nach einem der Ansprüche 1 bis 6,
**dadurch gekennzeichnet,**
**dass** die Zwischenverdampfung im zwei- oder mehrstufigen Reaktorsystem durch Verringerung des Drucks gegenüber dem Reaktionsdruck durchgeführt wird.

8. Verfahren nach einem der Ansprüche 1 bis 7,
**dadurch gekennzeichnet,**
**dass** bei der Verdampfung im zwei- oder mehrstufigen Reaktorsystem ein Wärmeaustausch zwischen dem der Verdampfung zugeführten flüssigen Strom und dem von der Verdampfung rückgeführten flüssigen Strom durchgeführt wird.

9. Verfahren nach einem der Ansprüche 1 bis 8,
**dadurch gekennzeichnet,**
**dass** das der Reaktoraustrag in Isobuten, Nebenprodukte, Wasser und ein TBA/Wasser-Gemisch mit einem Wassergehalt unter 10 Massen-%, das in den Reaktor zurückgefahren wird, getrennt wird.

10. Verfahren nach einem der Ansprüche 1 bis 9,
**dadurch gekennzeichnet,**
**dass** aus dem Reaktoraustrag in einer ersten Kolonne über Kopf der überwiegende Teil des Isobutens als Isobuten/Wasser-Azeotrop abgetrennt wird.

11. Verfahren nach einem der Ansprüche 1 bis 10,
**dadurch gekennzeichnet,**
**dass** aus dem Kopfprodukt der ersten Kolonne nach Abtrennung eines Teil des Wassers durch Phasentrennung durch azeotrope Destillation in einer zweiten Kolonne (16) ein nahezu wasserfreies Isobuten gewonnen wird.

12. Verfahren nach einem der Ansprüche 1 bis 11,
**dadurch gekennzeichnet,**
**dass** C₈-Kohlenwasserstoffe und langkettigere Kohlenwasserstoffe, die als Nebenprodukte im Reaktionsaustrag vorliegen aus dem isobutenfreien Sumpfprodukt der ersten Kolonne (4) in einer dritten Kolonne (6) als Kopfprodukt abgetrennt werden.

13. Verfahren nach einem der Ansprüche 1 bis 11,
**dadurch gekennzeichnet,**
**dass** die C₈-Kohlenwasserstoffe und eventuell langkettigere Kohlenwasserstoffe, die als Nebenprodukte im Reaktionsaustrag vorliegen, als Sumpfprodukt in einer vierten Kolonne (23), in der ein dampfförmiger oder flüssiger Seitenabzug der ersten Kolonne (4) destilliert wird, abgetrennt werden.

14. Verfahren nach einem der Ansprüche 1 bis 13,
**dadurch gekennzeichnet,**
**dass** aus dem Sumpfprodukt der ersten Kolonne (4) oder dritten Kolonne (6) in einer fünften Kolonne (9) über Kopf ein Gemisch aus Wasser und TBA abgetrennt wird, welches gegebenenfalls Isobuten aufweisen kann und als Sumpfprodukt Wasser, welches gegebenenfalls organische Verunreinigungen aufweist, wobei das tert.-Butanol/WasserGemisch zumindest teilweise in den Reaktor zur Spaltung des tert.-Butanols zurückgeführt wird.

15. Verfahren nach einem der Ansprüche 1 bis 14,
**dadurch gekennzeichnet,**
**dass** 2-Butanol destillativ aus dem als Sumpfprodukt abgetrennten Wasser oder als Seitenstrom in der fünften Kolonne (9) abgetrennt wird.

16. Verfahren nach einem der Ansprüche 1 bis 15,
**dadurch gekennzeichnet,**
**dass** das Destillat (12) einer Isobuten-Anreicherung (4a), welches im wesentlichen ein Isobuten/Wasser-Azeotrop aufweist, kondensiert und in einen Absitz- oder Trennbehälter überführt wird oder alternativ direkt in einen Absitz- oder Trennbehälter mit Wärmeaustauscherelementen kondensiert wird und dass nach der Flüssig-Flüssig-Trennung ein Teil der organischen Flüssigkeit einer Kolonne (16) am Kopf oder im oberen Teil der Kolonne zugeführt wird und in dieser nahezu wasserfreies Isobuten (18) im Sumpf gewonnen wird und ein Isobuten/Wasser-Azeotrop über Kopf dieser Kolonne abgetrennt und wieder dem Flüssig-Flüssig-Trennbehälter zugeführt wird.

17. Verfahren nach einem der Ansprüche 1 bis 16,
**dadurch gekennzeichnet,**
**dass** aus dem Reaktionsaustrag vor dem Auftrennen in verschiedene Fraktionen in einer ersten Kolonne ein Teil des im Reaktionsaustrag vorhandenen Wassers durch Pervaporation und/oder Dampfpermeation mittels einer Membran abgetrennt wird.

18. Verfahren nach einem der Ansprüche 1 bis 17,
**dadurch gekennzeichnet,**
**dass** aus dem Reaktionszulauf vor dem Eintritt in den Reaktor, also aus Strom (1), Strom (10) und/oder dem daraus gemischten Strom, ein Teil des vorhandenen Wassers durch Pervaporation und/oder Dampfpermeation mittels einer Membran abgetrennt wird.

19. Verfahren nach einem der Ansprüche 1 bis 18,
**dadurch gekennzeichnet,**
**dass** aus dem Reaktionsaustrag vor dem Auftrennen in verschiedene Fraktionen in einer ersten Kolonne ein Teil des im Reaktionsaustrag vorhandenen Wassers durch Abscheidung in einem Trenn- oder Absitzbehälter abgetrennt wird.

20. Verfahren nach Anspruch 19,
**dadurch gekennzeichnet,**
**dass** das gesamte Reaktionswasser bis auf den Teil, der gegebenenfalls für die destillative Abtrennung von Isobuten und C₈-Kohlenwasserstoffen durch Azeotropdestillation notwendig ist, abgetrennt wird.

21. Verfahren nach einem der Ansprüche 1 bis 20,
**dadurch gekennzeichnet,**
**dass** Isobuten in einer Reinheit von 99 Massen-% gewonnen wird, wobei der maximale Wassergehalt bei Werten kleiner 250 ppm liegt.

22. Reaktorsystem zur isothermen Durchführung von Gleichgewichtsreaktionen in der Flüssigphase an Festbettkatalysatoren, bei denen zumindest eines der Reaktionsprodukte unter Temperatur und Druckbedingungen, bei denen alle Edukte sowie zumindest ein Reaktionsprodukt überwiegend flüssig vorliegt, zum Teil gasförmig vorliegt, insbesondere zur Durchführung des Verfahren gemäß einem der Ansprüche 1 bis 21,
**dadurch gekennzeichnet,**
**dass** das Reaktorsystem zumindest zwei baulich voneinander getrennte Reaktionszonen aufweist, die jeweils einen im Festbett angeordneten Katalysator sowie Vorrichtungen zur isothermen Temperaturführung aufweisen, wobei zwischen zwei Reaktionszonen jeweils eine Abtrennzone vorhanden ist, die Mittel aufweist, mit denen zumindest ein Teil zumindest eines der Produkte der Gleichgewichtsreaktion durch Druck oder Temperaturänderung in die Gasphase überführt, vom Reaktionsgemisch abgetrennt und aus dem Reaktor ausgeschleust wird und mit denen das restliche Reaktionsgemisch in die folgende Reaktionszone überführt wird, wobei gegebenenfalls ein weiteres Mittel vorhanden sein kann, mit welchem die Temperatur oder der Druck in der folgenden Reaktionszone genau so eingestellt wird wie in der vorangegangenen Reaktionszone.

## Claims

1. Process for preparing isobutene by dissociation of tert-butanol into isobutene and water over a strong acid ion-exchange resin arranged as a fixed bed in at least one reactor at a temperature of from 80 to 150°C and a pressure of from 5 to 25 bar, and subsequent separation of the reaction mixture into isobutene, by-products, water and at least one mixture of undissociated tert-butanol and water, **characterized in that** the reactor is operated pseudoisothermally, with a temperature difference between the inflowing and outflowing streams of less than 15 K, and the process is carried out with the dissociation being carried out to the equilibrium of the dissociation reaction in a first reaction zone, isobutene being separated off in gaseous form in a subsequent separation zone and the dissociation being continued to the equilibrium of the dissociation reaction in a subsequent reaction zone, and, if further separation zones and reaction zones are present, these process steps being repeated the appropriate number of times.

2. Process according to Claim 1, **characterized in that** the feed to the reactor is composed of fresh feed stream and one or more recycle streams which comprise a mixture of undissociated tert-butanol, water and possibly by-products, with the mass ratio of fresh feed stream to the sum of the recycle streams being less than 1 : 10.

3. Process according to Claim 1 or 2, **characterized in that** a plurality of reactors connected in series or in parallel are used and all reactors are operated isothermally.

4. Process according to any of Claims 1 to 3, **characterized in that** at least one reactor having at least two reaction zones which each have a strong acid ion-exchange resin arranged as a fixed bed, where between each two reaction zones there is a separation zone in which at least part of one of the products of the dissociation is separated off from the reaction mixture by bringing it (partly) into the gas phase and is discharged from the reactor, is used.

5. Process according to any of Claims 1 to 4, **characterized in that** the pressure and the temperature in the reaction zones of the reactor are chosen so that the reaction mixture is present in the liquid phase and the pressure and the temperature in the separation zones are chosen so that part of the isobutene is present in the gas phase.

6. Process according to any of Claims 1 to 5, **characterized in that** the process is carried out in at least two reactors and the liquid reactor output from the first reactor is passed to a zone in which part of the reactor output is brought into a gas phase by changing the pressure and/or temperature and is separated off from the liquid phase and the liquid phase is passed to the second reactor.

7. Process according to any of Claims 1 to 6, **characterized in that** the intermediate vaporization in a two-stage or multistage reactor system is carried out by reducing the pressure to below the reaction pressure.

8. Process according to any of Claims 1 to 7, **characterized in that**, in the vaporization in a two-stage or multistage reactor system, heat exchange is carried out between the liquid stream fed to the vaporization and the liquid stream returned from the vaporization.

9. Process according to any of Claims 1 to 8, **characterized in that** the reactor output is separated into isobutene, by-products, water and a TBA/water mixture which has a water content of less than 10% by mass and is recirculated to the reactor.

10. Process according to any of Claims 1 to 9, **characterized in that** the major part of the isobutene is separated off from the reactor output as an isobutene/water azeotrope at the top of a first column.

11. Process according to any of Claims 1 to 10, **characterized in that** part of the water is separated off from the overhead product from the first column by phase separation and a virtually water-free isobutene is then isolated by azeotropic distillation in a second column (16).

12. Process according to any of Claims 1 to 11, **characterized in that** C₈-hydrocarbons and longer-chain hydrocarbons which are present as by-products in the output from the reactor are separated off from the isobute-free bottom product from the first column (4) as overhead product in a third column (6).

13. Process according to any of Claims 1 to 11, **characterized in that** the C₈-hydrocarbons and any longer-chain hydrocarbons which are present as by-products in the output from the reactor are separated off as bottom product in a fourth column (23) in which a gaseous or liquid side stream taken off from the first column (4) is distilled.

14. Process according to any of Claims 1 to 13, **characterized in that** the bottom product from the first column (4) or the third column (6) is separated off in a fifth column (9) into a mixture of water and TBA, which may contain isobutene, as overhead product and, as bottom product, water which may contain organic impurities, with the tert-butanol/water mixture being at least partly recirculated to the reactor for dissociation of tert-butanol.

15. Process according to any of Claims 1 to 14, **characterized in that** 2-butanol is separated off by distillation from the water which has been separated off as bottom product or is separated off as side stream in the fifth column (9).

16. Process according to any of Claims 1 to 15, **characterized in that** the distillate (12) from an isobutene enrichment (4a), which consists essentially of an isobutene/water azeotrope, is condensed and transferred to a settling or separation vessel or alternatively is condensed directly in a settling or separation vessel having heat-exchange elements and, after the liquid/liquid separation, part of the organic liquid is fed to a column (16) at the top or in the upper part of the column to give virtually water-free isobutene (18) at the bottom and an isobutene/water azeotrope at the top of this column, with the isobutene/water azeotrope being returned to the liquid/liquid separation vessel.

17. Process according to any of Claims 1 to 16, **characterized in that** part of the water present in the output from the reactor is separated off from the latter by pervaporation and/or vapor permeation by means of a membrane before the reactor output is separated into various fractions in a first column.

18. Process according to any of Claims 1 to 17, **characterized in that** part of the water present is separated off from the reactor feed prior to entry into the reactor, i.e. from stream (1), steam (10) and/or the resulting mixed stream, by pervaporation and/or vapor permeation by means of a membrane.

19. Process according to any of Claims 1 to 18, **characterized in that** part of the water present in the output from the reactor is separated off by separation in a separation or settling vessel before the output from the reactor is separated into various fractions in a first column.

20. Process according to Claim 19, **characterized in that** all of the water of reaction except for the part which may be necessary to allow isobutene and C₈-hydrocarbons to be separated off by azeotropic distillation is separated off.

21. Process according to any of Claims 1 to 20, **characterized in that** isobutene is obtained in a purity of 99% by mass, with the maximum water content being less than 250 ppm.

22. Reactor system for carrying out equilibrium reactions isothermally in the liquid phase over fixed-bed catalysts, where at least one of the reaction products is partly present in gaseous form under temperature and pressure conditions at which all starting materials and at least one reaction product are predominantly present in liquid form, in particular for carrying out the process according to any of Claims 1 to 21, **characterized in that** the reactor system has at least two physically separate reaction zones which each have a catalyst arranged in a fixed bed and facilities for maintaining isothermal operation, where between each two reaction zones there is a separation zone having means for bringing at least part of at least one of the products of the equilibrium reaction into the gas phase by changing the pressure or temperature, separating it off from the reaction mixture and discharging it from the reactor and for transferring the remaining reaction mixture to the following reaction zone, with a further means which enables the temperature or pressure in the subsequent reaction zone to be set to precisely the value in the preceding reaction zone being able to be present if desired.

## Revendications

1. Procédé pour la production d'isobutène par coupure de tert-butanol en isobutène et eau dans au moins un réacteur sur une résine échangeuse d'ions fortement acide disposée en lit fixe dans ce réacteur, à une température de 80 à 150 °C et sous une pression de 5 à 25 bars et fractionnement subséquent du mélange réactionnel en isobutène, produits secondaires, eau et au moins un mélange de tert-butanol non dissocié et eau,
**caractérisé en ce que**
le réacteur est utilisé en mode quasi-isothermique avec une différence de température de moins de 15 K entre les courants entrant et sortant, et on effectue le procédé de manière que la coupure dans la première zone de réaction soit effectuée jusqu'à l'équilibre de la réaction de coupure, dans une zone de séparation subséquente de l'isobutène est séparé à l'état gazeux et dans une zone de réaction y faisant suite la coupure est à nouveau effectuée jusqu'à l'équilibre de la réaction de coupure, et, si d'autres zones de séparation et zones de réaction sont présentes, ces étapes de processus sont répétées un nombre de fois correspondant.

2. Procédé selon la revendication 1,
**caractérisé en ce que**
le courant d'alimentation arrivant au réacteur se compose d'un courant de produit de départ neuf et d'un ou plusieurs courants de recyclage comportant un mélange de tert-butanol non dissocié, eau et éventuellement produits secondaires, le rapport massique du courant de produit de départ neuf à la somme des courants de recyclage étant inférieur à 1 : 10.

3. Procédé selon la revendication 1 ou 2, **caractérisé**
**en ce qu'**on utilise plusieurs réacteurs qui sont disposés en série ou en parallèle, en faisant fonctionner tous les réacteurs en mode isothermique.

4. Procédé selon l'une quelconque des revendications 1 à 3,
**caractérisé**
**en ce qu'**on utilise au moins un réacteur qui comporte au moins deux zones de réaction qui comportent chacune une résine échangeuse d'ions fortement acide disposée en lit fixe, entre deux zones de réaction étant chaque fois présente une zone de séparation, dans laquelle au moins une partie de l'un des produits de la coupure est séparée du mélange réactionnel par transfert dans la phase gazeuse et évacuée du réacteur.

5. Procédé selon l'une quelconque des revendications 1 à 4,
**caractérisé**
**en ce qu'**on choisit la pression et la température dans les zones de réaction du réacteur de manière que le mélange réactionnel se trouve en phase liquide, et on choisit la pression et la température dans les zones de séparation de manière que l'isobutène se trouve en partie dans la phase gazeuse.

6. Procédé selon l'une quelconque des revendications 1 à 5,
**caractérisé**
**en ce qu'**on effectue le procédé dans au moins deux réacteurs, la décharge liquide de réacteur du premier réacteur étant transférée dans une zone dans laquelle une partie de la décharge de réacteur est convertie, par modification de la pression et/ou de la température, en une phase gazeuse et est séparée de la phase liquide et la phase liquide est transférée dans le deuxième réacteur.

7. Procédé selon l'une quelconque des revendications 1 à 6,
**caractérisé en ce que**
dans le système de réacteurs à deux ou plus de deux étages on effectue la vaporisation intermédiaire par abaissement de la pression par rapport à la pression de la réaction.

8. Procédé selon l'une quelconque des revendications 1 à 7,
**caractérisé en ce que**
dans la vaporisation dans le système de réacteurs à deux ou plus de deux étages un échange de chaleur est effectué entre le courant liquide envoyé à la vaporisation et le courant liquide renvoyé de la vaporisation.

9. Procédé selon l'une quelconque des revendications 1 à 8,
**caractérisé en ce que**
la décharge de réacteur est fractionnée en isobutène, produits secondaires, eau et mélange de TBA/eau ayant une teneur en eau inférieure à 10 % en masse, qui est recyclé dans le réacteur.

10. Procédé selon l'une quelconque des revendications 1 à 9,
**caractérisé en ce que**
la majeure partie de l'isobutène est séparée de la décharge du réacteur sous forme d'azéotrope isobutène/eau par la tête dans une première colonne.

11. Procédé selon l'une quelconque des revendications 1 à 10,
**caractérisé**
**en ce qu'**à partir du produit de tête de la première colonne, après séparation d'une partie de l'eau par séparation de phases, on obtient par distillation azéotropique dans une deuxième colonne (16) un isobutène pratiquement anhydre.

12. Procédé selon l'une quelconque des revendications 1 à 11,
**caractérisé en ce que**
dans une troisième colonne (6) des hydrocarbures en C₈ et des hydrocarbures à plus longue chaîne, qui sont présents en tant que produits secondaires dans la décharge de la réaction, sont séparés en tant que produit de tête d'avec le produit de pied exempt d'isobutène de la première colonne (4).

13. Procédé selon l'une quelconque des revendications 1 à 11,
**caractérisé en ce que**
les hydrocarbures en C₈ et éventuellement les hydrocarbures à plus longue chaîne, qui sont présents en tant que produits secondaires dans la décharge de la réaction, sont séparés en tant que produit de pied dans une quatrième colonne (23) dans laquelle est distillée une décharge latérale liquide ou sous forme de vapeur de la première colonne (4).

14. Procédé selon l'une quelconque des revendications 1 à 13,
**caractérisé**
**en ce qu'**à partir du produit de pied de la première colonne (4) ou troisième colonne (6) est séparé par la tête dans une cinquième colonne (9) un mélange d'eau et de TBA, qui peut éventuellement comporter de l'isobutène, et en tant que produit de pied de l'eau, qui éventuellement comporte des impuretés organiques, le mélange tert-butanol/eau étant au moins partiellement recyclé dans le réacteur pour la coupure du tert-butanol.

15. Procédé selon l'une quelconque des revendications 1 à 14,
**caractérisé en ce que**
du 2-butanol est séparé par distillation d'avec l'eau séparée en tant que produit de pied ou en tant que courant latéral dans la cinquième colonne (9).

16. Procédé selon l'une quelconque des revendications 1 à 15,
**caractérisé en ce que**
le distillat (12) d'un enrichissement en isobutène (4a), qui comporte essentiellement un azéotrope isobutène/eau, est condensé et transféré dans un récipient de décantation ou de séparation ou bien directement condensé dans un récipient de décantation ou de séparation comportant des éléments échangeurs de chaleur et **en ce qu'**après la séparation liquide-liquide une partie du liquide organique est envoyée à une colonne (16) à la tête ou dans la partie supérieure de la colonne et dans celle-ci on obtient par le pied de l'isobutène (18) pratiquement anhydre et un azéotrope isobutène/eau est séparé par la tête de cette colonne et envoyé de nouveau au récipient de séparation liquide-liquide.

17. Procédé selon l'une quelconque des revendications 1 à 16,
**caractérisé**
**en ce qu'**à partir de la décharge de réaction avant le fractionnement en diverses fractions dans une première colonne une partie de l'eau présente dans la décharge de réaction est séparée par pervaporation et/ou perméation de vapeur au moyen d'une membrane.

18. Procédé selon l'une quelconque des revendications 1 à 17,
**caractérisé**
**en ce qu'**une partie de l'eau présente est séparée du courant d'alimentation de la réaction avant l'entrée dans le réacteur, c'est-à-dire du courant (1), du courant (10) et/ou du courant consistant en un mélange de ceux-ci, par pervaporation et/ou perméation de vapeur au moyen d'une membrane.

19. Procédé selon l'une quelconque des revendications 1 à 18,
**caractérisé**
**en ce qu'**à partir de la décharge de réaction avant le fractionnement en diverses fractions dans une première colonne une partie de l'eau présente dans la décharge de réaction est séparée par séparation dans un récipient de séparation ou décantation.

20. Procédé selon la revendication 19,
**caractérisé**
**en ce qu'**on sépare la totalité de l'eau de réaction jusqu'à la partie qui est éventuellement requise pour la séparation par distillation d'isobutène et d'hydrocarbures en C₈ par distillation azéotropique.

21. Procédé selon l'une quelconque des revendications 1 à 20,
**caractérisé**
**en ce qu'**on obtient de l'isobutène à un degré de pureté de 99 % en masse, la teneur maximale en eau se situant à des valeurs inférieures à 250 ppm.

22. Système de réacteurs pour l'exécution isothermique de réactions d'équilibre dans la phase liquide de catalyseurs en lit fixe, dans lequel au moins l'un des produits de réaction se trouve en partie à l'état gazeux dans les conditions de température et de pression dans lesquelles tous les produits de départ ainsi qu'au moins un produit de réaction se trouvent essentiellement à l'état liquide, en particulier pour la mise en oeuvre du procédé selon l'une quelconque des revendications 1 à 21,
**caractérisé en ce que** le système de réacteurs comporte au moins deux zones de réaction séparées l'une de l'autre par construction, qui comportent chacune un catalyseur disposé en lit fixe ainsi que des dispositifs pour la conduite isothermique de la température, entre deux zones de réaction étant présente chaque fois une zone de séparation qui comporte des moyens à l'aide desquels au moins une partie d'au moins l'un des produits de la réaction d'équilibre est convertie en la phase gazeuse par modification de la pression ou de la température, séparée du mélange réactionnel et évacuée du réacteur et à l'aide desquels le mélange réactionnel restant est transféré dans la zone de réaction suivante, éventuellement étant présent un autre moyen à l'aide duquel la température ou la pression dans la zone de réaction suivante est réglée exactement comme dans la zone de réaction précédente.
